# EUROPEAN PATENT SPECIFICATION

(11) **EP 3 923 808 B1**
(45) Date of publication and mention of the grant of the patent: **07.06.2023**
(21) Application number: 20713421.4
(22) Date of filing: 11.02.2020
(51) Int. Cl.: A61B 5/15, A61B 5/151

(54) **CAPILLARY COLLECTOR WITH ROTATABLE CONNECTION**
KAPILLARSAMMLER MIT DREHBARER VERBINDUNG
COLLECTEUR CAPILLAIRE À CONNEXION ROTATIVE

(30) Priority: 14.02.2019 US 201962805398 P
(43) Date of publication of application: 22.12.2021
(60) Divisional application: 23153538.6
(73) Proprietor: Becton, Dickinson and Company, Franklin Lakes, NJ 07417 (US)
(72) Inventor: YAKHNICH, Vlad, Park Ridge, New Jersey 07656 (US); BOKKA SRINIVASA RAO, Kishore K., Ridgewood, New Jersey 07450 (US); TORRIS, Anthony, V., Montclair, New Jersey 07042 (US); IVOSEVIC, Milan, Kinnelon, New Jersey 07405 (US); EDELHAUSER, Adam, Kinnelon, New Jersey 07405 (US)
(74) Representative: Germain Maureau
(86) International application number: PCT/US2020/017635
(87) International publication number: WO 2020/167746

(56) References cited:
- WO-A1-2014/172238
- WO-A1-2018/039307
- JP-A- 2002 219 115
- US-A1- 2011 118 568
- US-A1- 2014 257 066
- US-B2- 9 380 975

## Description

### BACKGROUND OF THE INVENTION

### Field of the Disclosure

The present disclosure relates generally to a device for obtaining a biological sample. More particularly, the present disclosure relates to an integrated finger-based capillary blood collection device with the ability to lance and squeeze a finger, collect, stabilize, and dispense a blood sample in a controlled manner.

### Description of the Related Art

Devices for obtaining and collecting biological samples, such as blood samples, are commonly used in the medical industry. One type of blood collection that is commonly done in the medial field is capillary blood collection which is often done to collect blood samples for testing. Certain diseases, such as diabetes, require that the patient's blood be tested on a regular basis to monitor, for example, the patient's blood sugar levels. Additionally, test kits, such as cholesterol test kits, often require a blood sample for analysis. The blood collection procedure usually involves pricking a finger or other suitable body part in order to obtain the blood sample. Typically, the amount of blood needed for such tests is relatively small and a small puncture wound or incision normally provides a sufficient amount of blood for these tests. Various types of lancet devices have been developed which are used for puncturing the skin of a patient to obtain a capillary blood sample from the patient.

Many different types of lancet devices are commercially available to hospitals, clinics, doctors' offices, and the like, as well as to individual consumers. Such devices typically include a sharp-pointed member such as a needle, or a sharp-edged member such as a blade, that is used to make a quick puncture wound or incision in the patient's skin in order to provide a small outflow of blood. It is often physiologically and psychologically difficult for many people to prick their own finger with a hand-held needle or blade. As a result, lancet devices have evolved into automatic devices that puncture or cut the skin of the patient upon the actuation of a triggering mechanism. In some devices, the needle or blade is kept in a standby position until it is triggered by the user, who may be a medical professional in charge of drawing blood from the patient, or the patient himself or herself. Upon triggering, the needle or blade punctures or cuts the skin of the patient, for example, on the finger. Often, a spring is incorporated into the device to provide the "automatic" force necessary to puncture or cut the skin of the patient.

One type of contact activated lancet device that features automatic ejection and retraction of the puncturing or cutting element from and into the device is U.S. Patent No. 9,380,975, which is owned by Becton, Dickinson and Company, the assignee of the present application. This lancet device includes a housing and a lancet structure having a puncturing element. The lancet structure is disposed within the housing and adapted for movement between a retaining or pre-actuated position wherein the puncturing element is retained within the housing, and a puncturing position wherein the puncturing element extends through a forward end of the housing. The lancet device includes a drive spring disposed within the housing for biasing the lancet structure toward the puncturing position, and a retaining hub retaining the lancet structure in the retracted position against the bias of the drive spring. The retaining hub includes a pivotal lever in interference engagement with the lancet structure. An actuator within the housing pivots the lever, thereby moving the lancet structure toward the rearward end of the housing to at least partially compress the drive spring, and releasing the lever from interference engagement with the lancet structure. The blood sample that is received is then collected and/or tested. This testing can be done by a Point-of-Care (POC) testing device or it can be collected and sent to a testing facility.

Currently, capillary blood collection workflow is a complex multi-step process requiring high skill level. The multi-step nature of this process introduces several variables that could cause sample quality issues such as hemolysis, inadequate sample stabilization, and micro-clots. The use of lancet devices for obtaining blood samples can result in several variables that effect the collection of the capillary blood sample, including, but not limited to, holding the lancet still during the testing, obtaining sufficient blood flow from the puncture site, adequately collecting the blood, preventing clotting, and the like. Some of the most common sources of process variability are: (1) inadequate lancing site cleaning and first drop removal which can potentially result in a contaminated sample; (2) inconsistent lancing location and depth which could potentially result in insufficient sample volume and a large fraction of interstitial fluid; (3) inconsistent squeezing technique and excessive pressure near the lancing site to promote blood extraction (e.g., blood milking) which could potentially result in a hemolyzed sample; (4) variable transfer interfaces and collection technique which could potentially result in a hemolyzed or contaminated sample; and (5) inadequate sample mixing with anticoagulant which could potentially result in micro-clots.

Thus, there is a need in the art for a device that has the ability to lance and squeeze the finger, collect the sample, stabilize the sample, and subsequently dispense the sample in a controlled manner. There is also a need in the art for a device that simplifies and streamlines the capillary blood collection by eliminating workflow variabilities which are typically associated with low sample quality including hemolysis and micro-clots. There is still a further need in the art for a closed system collection and transfer that eliminate blood exposure and device reuse. There is still a further need in the art for a device that: (1) introduces flexibility in the accommodation of different capillary blood collection and transfer container; (2) has the capability to generate high quality uniformly mixed/stabilized capillary blood samples; (3) has the capability to generate on-board plasma from capillary plasma samples; (4) has the capability to collect large capillary blood samples (> 50-500µL) at reduced pain; (5) contains a unique sample identifier that is paired with patient information at the time of collection; (6) has the capability to collect capillary blood and perform on-board diagnostics; and (7) has multiple collection ports to collect a blood sample into different containers having the same or different anticoagulants.

### SUMMARY OF THE INVENTION

The present disclosure is directed to a device for obtaining a biological sample, such as a capillary blood collection device, which meets the needs set forth above and has the ability to lance and squeeze the finger, collect the sample, stabilize the sample, and subsequently dispense the sample in a controlled manner. The device also simplifies and streamlines the capillary blood collection by eliminating workflow variabilities which are typically associated with low sample quality including hemolysis and micro-clots.

The present disclosure includes a self-contained and fully integrated finger-based capillary blood collection device with ability to lance, collect and stabilize high volume capillary blood sample, e.g., up to or above 500 microliters. The device simplifies and streamlines high volume capillary blood collection by eliminating workflow steps and variabilities which are typically associated with low sample quality including hemolysis, micro-clots, and patient discomfort. The device comprises a retractable lancing mechanism that can lance the finger and an associated blood flow path which ensures attachment and transfer of the capillary blood from the pricked finger site to the collection container. The device also includes a holder that can be cyclically squeezed to stimulate, i.e., pump, blood flow out of the finger and also anticoagulant deposited in the flow path or collection container to stabilize collected sample.

According to one design, the device can comprise discrete components such as a holder, a lancet, and a collection container. According to another design, the lancet and collection container can be integrated into one device which is then used with the holder. According to yet another design, the holder, lancet, and collection container can be integrated into a single system. Any of these designs are envisioned to be used as a self-standing disposable device and/or in association with an external power source for pain reduction control. The capillary blood collection device can serve as a platform for various capillary blood collection containers ranging from small tubes to capillary dispensers, as well as on-board plasma separation modules. This capability extends the product flexibility to various applications including dispensing to a Point-of-Care (POC) cartridge or to a small collection tube transfer which can be used in a centrifuge or an analytical instrument.

In accordance with the present invention, a device for obtaining a blood sample comprises a holder for receiving a sample source, the holder having an actuation portion and a port; a container engagement portion connected to the holder; and a collection container removably connectable to the container engagement portion, wherein the container engagement portion allows the collection container to rotate between a first position in which the collection container is spaced from the port and a second position in which the collection container is in fluid communication with the port.

In accordance with an embodiment of the present invention, the actuation portion may be transitionable between a first position in which the holder defines a first ellipse and a second position in which the holder defines a second ellipse, wherein the second ellipse is different than the first ellipse. The actuation portion may include a contact member. The actuation portion may be transitionable between a first position in which the contact member is in a disengaged position and a second position in which the contact member is in an engaged position. With the contact member in the engaged position, the contact member may exert a pressure on the sample source. The actuation portion may include a pumping member for applying pressure to the sample source. The pumping member may include a pair of opposed tabs. The sample source may be a finger. With the finger received within the holder, the port may be in communication with a portion of the finger. The container engagement portion and the holder may be connected via a ball joint, a peg and hole pivot joint, a pin and C-clip pivot joint, or a pin and pocket pivot j oint. The holder may include a living hinge that is connectable to the container engagement portion. The living hinge may be connected to the container engagement portion via a keyed snap-fit arrangement, a hook and snap-fit arrangement, a C-clip and snap-fit arrangement, or a peg and profiled engagement. The collection container may be removably connectable to the container engagement portion, the container defining a collection cavity.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**Fig. 1** is a perspective view of a holder in accordance with an embodiment of the present invention.
**Fig. 2** is a perspective view of a holder in a first position in accordance with an embodiment of the present invention.
**Fig. 3** is a perspective view of a holder in a second position in accordance with an embodiment of the present invention.
**Fig. 4A** is a perspective view of a holder in accordance with another embodiment of the present invention.
**Fig. 4B** is a perspective view of a holder in accordance with another embodiment of the present invention.
**Fig. 5** is a perspective view of a holder in accordance with another embodiment of the present invention.
**Fig. 6A** is a perspective view of a holder in accordance with another embodiment of the present invention.
**Fig. 6B** is a perspective view of a holder in accordance with another embodiment of the present invention.
**Fig. 7A** is a perspective view of a holder in accordance with another embodiment of the present invention.
**Fig. 7B** is a perspective view of a holder in accordance with another embodiment of the present invention.
**Fig. 8** is an exploded, perspective view of a device having discrete components for obtaining a blood sample in accordance with an embodiment of the present invention.
**Fig. 9** is a perspective view of a holder with a lancet housing secured within a port in accordance with an embodiment of the present invention.
**Fig. 10** is a perspective view of a holder with a container secured within a port in accordance with an embodiment of the present invention.
**Fig. 11** is a perspective view of a semi-integrated device for obtaining a blood sample with an at-angle flow in accordance with another embodiment of the present invention.
**Fig. 12** is a perspective view of a holder with a lancet housing and container secured within a port in accordance with another embodiment of the present invention.
**Fig. 13** is a cross-sectional view of the device of **Fig. 12** in accordance with another embodiment of the present invention.
**Fig. 14** is a perspective view of a semi-integrated device for obtaining a blood sample with an in-line flow in accordance with another embodiment of the present invention.
**Fig. 15** is a perspective view of a holder with a lancet housing and container secured within a port in accordance with another embodiment of the present invention.
**Fig. 16** is a cross-sectional view of the device of **Fig. 15** in accordance with another embodiment of the present invention.
**Fig. 17** is a perspective view of an integrated device for obtaining a blood sample with an at-angle flow in accordance with another embodiment of the present invention.
**Fig. 18** is a cross-sectional view of the device of **Fig. 17** in accordance with another embodiment of the present invention.
**Fig. 19** is a cross-sectional view of the device of **Fig. 17** showing a blood flow path in accordance with another embodiment of the present invention.
**Fig. 20** is a perspective view of an integrated device for obtaining a blood sample with an in-line flow in accordance with another embodiment of the present invention.
**Fig. 21** is a cross-sectional view of the device of **Fig. 20** in accordance with another embodiment of the present invention.
**Fig. 22** is a cross-sectional view of the device of **Fig. 20** showing a blood flow path in accordance with another embodiment of the present invention.
**Fig. 23** is a perspective view of a first step of using an integrated device of the present disclosure in accordance with an embodiment of the present invention.
**Fig. 24** is a perspective view of a second step of using an integrated device of the present disclosure in accordance with an embodiment of the present invention.
**Fig. 25** is a perspective view of a third step of using an integrated device of the present disclosure in accordance with an embodiment of the present invention.
**Fig. 26** is a perspective view of a fourth step of using an integrated device of the present disclosure in accordance with an embodiment of the present invention.
**Fig. 27** is a perspective view of a first step of using a device having discrete components of the present disclosure in accordance with another embodiment of the present invention.
**Fig. 28** is a perspective view of a second step of using a device having discrete components of the present disclosure in accordance with another embodiment of the present invention.
**Fig. 29** is a perspective view of a third step of using a device having discrete components of the present disclosure in accordance with another embodiment of the present invention.
**Fig. 30** is a perspective view of a fourth step of using a device having discrete components of the present disclosure in accordance with another embodiment of the present invention.
**Fig. 31** is a perspective view of a fifth step of using a device having discrete components of the present disclosure in accordance with another embodiment of the present invention.
**Fig. 32** is an assembly perspective view of a device according to another embodiment of the present invention.
**Fig. 33** is another assembly perspective view of the device of **Fig. 32****.**
**Fig. 34a** is a perspective view of a connection arrangement between a connection interface and a container engagement portion according to one embodiment of the present invention.
**Fig. 34b** is a perspective view of a connection arrangement between a connection interface and a container engagement portion according to one embodiment of the present invention.
**Fig. 34c** is a perspective view of a connection arrangement between a connection interface and a container engagement portion according to one embodiment of the present invention.
**Fig. 34d** is a perspective view of a connection arrangement between a connection interface and a container engagement portion according to one embodiment of the present invention.
**Fig. 35** is an assembly perspective view of a device according to one embodiment of the present invention.
**Fig. 36** is another assembly perspective view of the device of **Fig. 35****.**
**Fig. 37a** is a perspective view of a connection arrangement between a connection interface and a container engagement portion according to one embodiment of the present invention.
**Fig. 37b** is a perspective view of a connection arrangement between a connection interface and a container engagement portion according to one embodiment of the present invention.
**Fig. 37c** is a perspective view of a connection arrangement between a connection interface and a container engagement portion according to one embodiment of the present invention.
**Fig. 37d** is a perspective view of a connection arrangement between a connection interface and a container engagement portion according to one embodiment of the present invention.
**Fig. 38** is a perspective view of a device according to the one embodiment of the present invention in a pre-activated position.
**Fig. 39** is a perspective view of the device of **Fig. 38** with a lancet inserted into a patient's finger.
**Fig. 40** is a perspective view of the device of **Fig. 38** showing rotation of a collection container into connection with a holder of the device.
**Fig. 41** is a perspective view of the device of **Fig. 38** showing collection of a blood sample from the patient's finger into the collection container.
**Fig. 42** is a perspective view of the device of **Fig. 38** showing removal of the collection container from the holder of the device.
**Fig. 43** is an illustration of a tray used to hold a device according to one embodiment of the present invention and a method for sizing and using a device held in the tray.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

The present disclosure is directed to a device for obtaining a biological sample, such as a capillary blood collection device, which meets the needs set forth above and has the ability to lance and squeeze the finger, collect the sample, stabilize the sample, and subsequently dispense the sample in a controlled manner. The device also simplifies and streamlines the capillary blood collection by eliminating workflow variabilities which are typically associated with low sample quality including hemolysis and micro-clots.

The present disclosure includes a self-contained and fully integrated finger-based capillary blood collection device with ability to lance, collect and stabilize high volume capillary blood sample, e.g., up to or above 500 microliters. The device simplifies and streamlines high volume capillary blood collection by eliminating workflow steps and variabilities which are typically associated with low sample quality including hemolysis, micro-clots, and patient discomfort. The device comprises a retractable lancing mechanism that can lance the finger and an associated blood flow path which ensures attachment and transfer of the capillary blood from the pricked finger site to the collection container. The device also includes a holder that can be cyclically squeezed to stimulate, i.e., pump, blood flow out of the finger and also anticoagulant deposited in the flow path or collection container to stabilize collected sample.

According to one design, the device can comprise discrete components such as a holder, a lancet, and a collection container. According to another design, the lancet and collection container can be integrated into one device which is then used with the holder. According to yet another design, the holder, lancet, and collection container can be integrated into a single system. Any of these designs are envisioned to be used as a self-standing disposable device and/or in association with an external power source for pain reduction control. The capillary blood collection device can serve as a platform for various capillary blood collection containers ranging from small tubes to capillary dispensers, as well as on-board plasma separation modules. This capability extends the product flexibility to various applications including dispensing to a Point-of-Care (POC) cartridge or to a small collection tube transfer which can be used in a centrifuge or an analytical instrument.

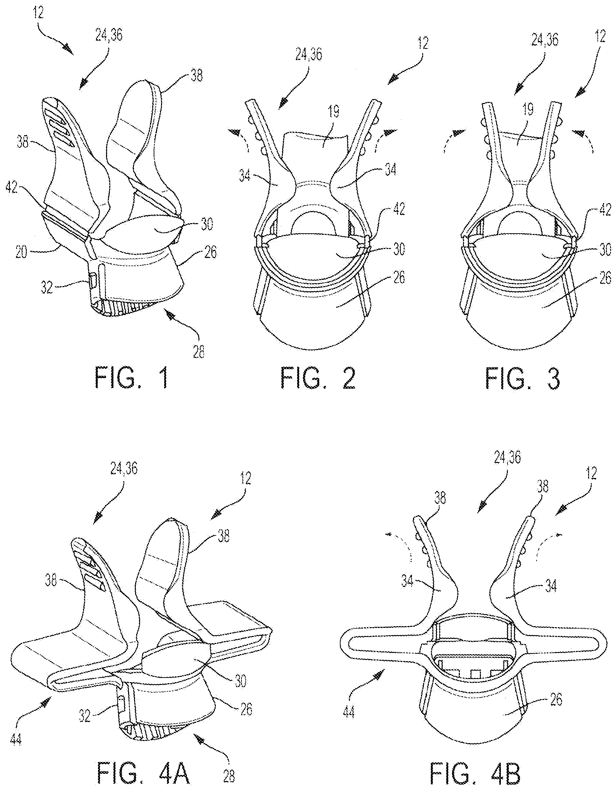

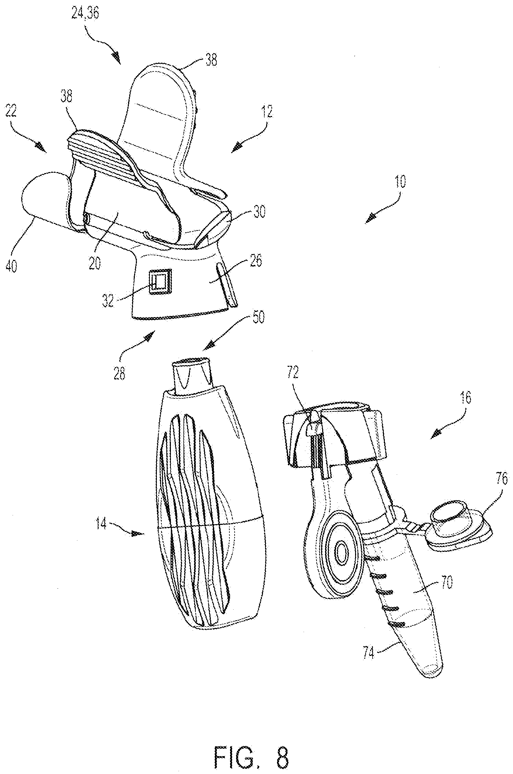

Referring to Figs. 8-10, in an exemplary embodiment, a device 10 of the present disclosure includes discrete components, e.g., a holder 12 (as shown in Figs. 1-7B), a lancet housing or lancet 14, and a collection container 16.

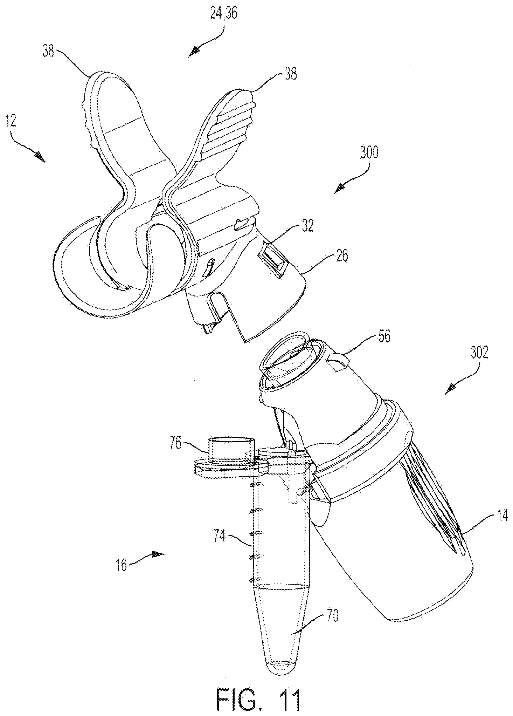

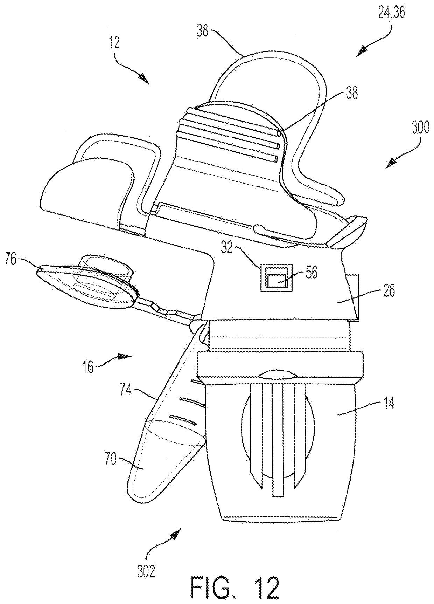

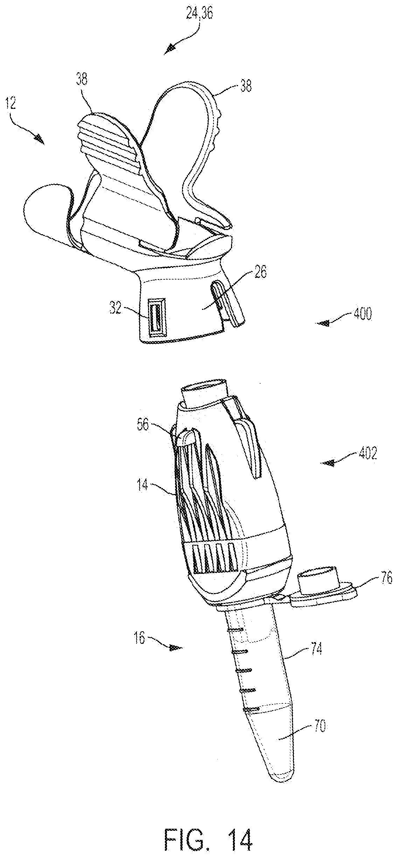

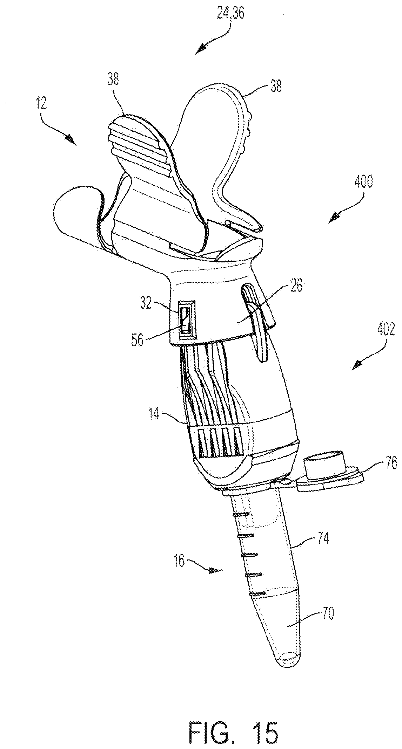

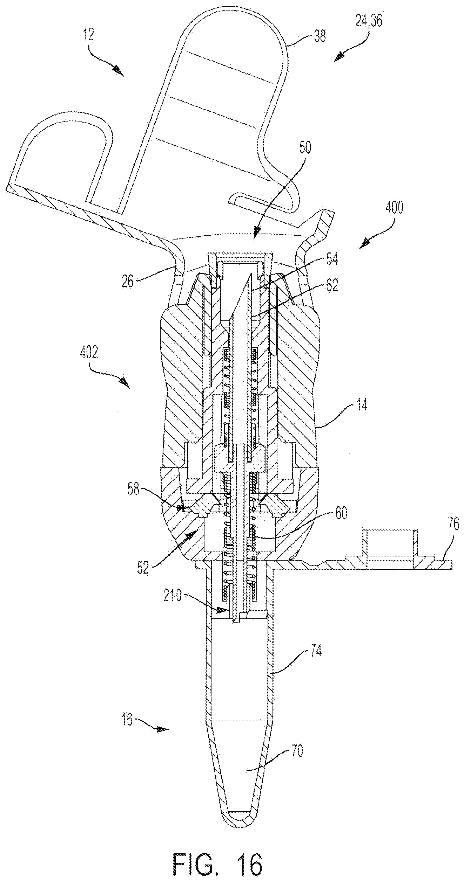

Referring to Figs. 11-13, in another exemplary embodiment, a semi-integrated device 300 of the present disclosure has an at-angle flow and includes an integrated lancet housing and collection container which can be connected with a separate holder. Referring to Figs. 14-16, in another exemplary embodiment, a semi-integrated device 400 of the present disclosure has an in-line flow and includes an integrated lancet housing and collection container which can be connected with a separate holder.

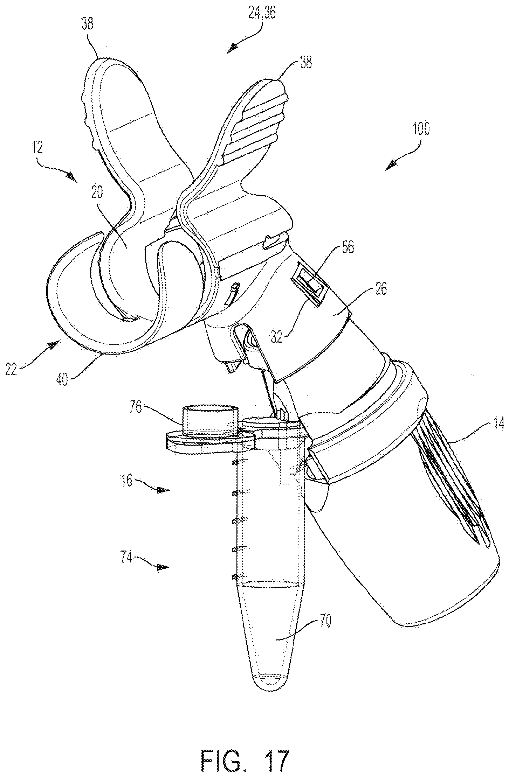

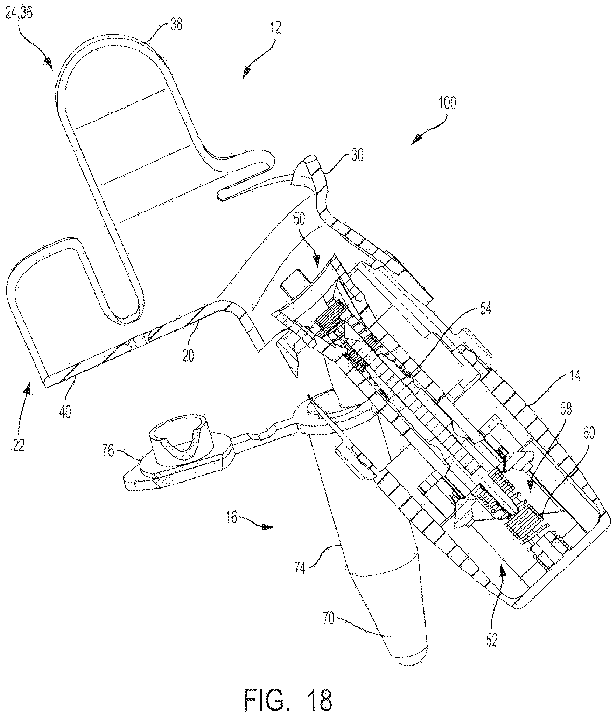

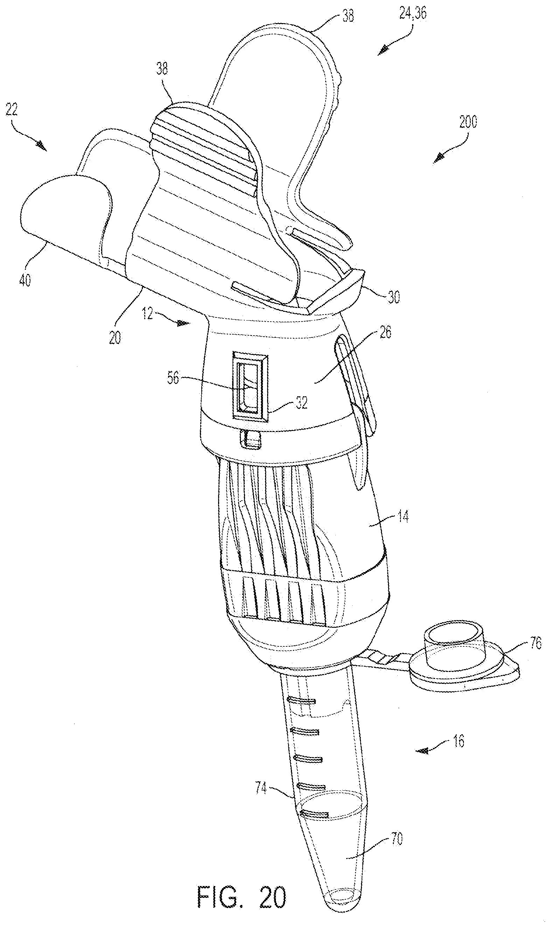

Referring to Figs. 17-19, in another exemplary embodiment, an integrated device 100 of the present disclosure has an at-angle flow and includes an integrated holder, lancet housing, and collection container. Referring to Figs. 20-22, in another exemplary embodiment, an integrated device 200 of the present disclosure has an in-line flow and includes an integrated holder, lancet housing, and collection container.

Referring to Figs. 8-10, in an exemplary embodiment, a device 10 for obtaining a blood sample 18 includes separate components, e.g., a holder 12, a lancet housing or lancet 14, and a collection container 16. Figs. 1-7B illustrate exemplary embodiments of a holder or finger housing 12 of the present disclosure.

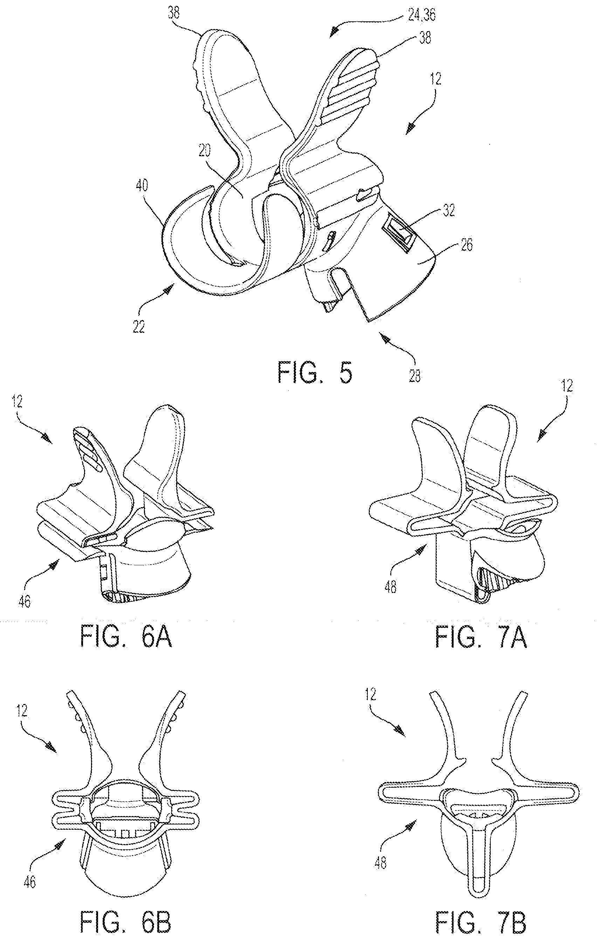

Referring to Figs. 1-7B, exemplary embodiments of holders 12 of the present disclosure that are able to receive a sample source, e.g., a finger 19, for supplying a biological sample, such as a blood sample 18. A holder 12 of the present disclosure generally includes a finger receiving portion 20 having a first opening 22 (Fig. 5), an actuation portion 24, a port 26 having a second opening 28, and a finger end guard 30. In one embodiment, the finger end guard 30 provides a stop portion for properly aligning and securing a finger 19 within the holder 12.

The first opening 22 of the finger receiving portion 20 is configured for receiving a sample source, e.g., a finger 19, for supplying a biological sample, such as a blood sample 18. It can be appreciated that the sample source could include other parts of the body capable of fitting within the first opening 22. The port 26 is in communication with the finger receiving portion 20. For example, with a finger 19 received within the holder 12, the port 26 is in communication with a portion of the finger 19. A holder 12 of the present disclosure can be sized to accommodate all finger sizes.

The second opening 28 of the port 26 is configured for receiving a lancet housing 14 and a collection container 16 as described in more detail below. In one embodiment, the port 26 includes a locking portion 32 for securely receiving the lancet housing 14 and the collection container 16 within the port 26.

In one embodiment, the actuation portion 24 is transitionable between a first position (Fig. 2) in which the holder 12 defines a first diameter and a second position (Fig. 3) in which the holder 12 defines a second diameter, wherein the second diameter is less than the first diameter. In one embodiment, the actuation portion 24 is transitionable between a first position (Fig. 2) in which the holder 12 defines a first elliptical shape, and a second position (Fig. 3) in which the holder 12 defines a second elliptical shape, wherein the first elliptical shape is different than the second elliptical shape. In this manner, with the holder 12 in the second position with a reduced diameter, a portion of the holder 12 contacts the sample source and the actuation portion 24 of the holder 12 is able to pump and/or extract blood 18 as described in more detail below.

Referring to Figs. 2 and 3, in one embodiment, the actuation portion 24 includes a contact member 34. Referring to Fig. 2, with the actuation portion 24 in the first position, the contact member 34 is in a disengaged position, i.e., the contact member 34 is provided in a first position with respect to a sample source, e.g., the finger 19, such that the contact member 34 may be in slight contact therewith. Referring to Fig. 3, with the actuation portion 24 in the second position, the contact member 34 is in an engaged position, i.e., the contact member 34 is provided in a second position with respect to the sample source, e.g., the finger 19, such that the contact member 34 is in an applied pressure contact with the finer 19, and the actuation portion 24 of the holder 12 is able to pump and/or extract blood 18. For example, with the contact member 34 in the engaged position, the contact member 34 exerts a pressure on the sample source.

Referring to Figs. 2 and 3, in one embodiment, the actuation portion 24 includes a pumping member 36 for applying pressure to the sample source, e.g., the finger 19. In one embodiment, the pumping member 36 comprises a pair of opposed tabs or wings 38. In such an embodiment, each tab 38 may include a contact member 34. Referring to Figs. 1-3, in one embodiment, the holder 12 includes a living hinge portion 42. The living hinge portion 42 allows a user to squeeze the wings 38 between a first position (Fig. 2) and a second position (Fig. 3).

Advantageously, the holder 12 of the present disclosure allow a user to repeatedly squeeze and release the wings 38 to pump and/or extract blood 18 from a finger 19 until a desired amount of blood 18 is filled in a collection container 16.

Advantageously, with the holder 12 placed onto a finger 19, the holder 12 does not constrict the blood flow and defines lancing and finger squeezing locations. The squeezing tabs or wings 38 provide a pre-defined range of squeezing pressure that is consistently applied throughout a finger 19. By doing so, the holder 12 provides a gentle controlled finger massage that stimulates blood extraction and minimizes any potential hemolysis.

Referring to Fig. 5, in one embodiment, the holder 12 includes a stability extension portion 40. This provides additional support for the holder 12 to be securely placed onto a finger 19. In one embodiment, the finger receiving portion 20 forms a generally C-shaped member and includes a plurality of inner gripping members for providing additional grip and support for the holder 12 to be securely placed onto a finger 19.

In one embodiment, the finger receiving portion 20 is formed of a flexible material. In some embodiments, the finger receiving portion 20 and the port 26 are formed from a flexible material.

Figs. 4A-7B illustrate other exemplary embodiments of the holder 12 of the present disclosure. Referring to Figs. 4A-4B, the holder 12 includes a leaf spring 44. Referring to Figs. 6A-6B, the holder 12 includes a double leaf spring 46. Referring to Figs. 7A-7B, the holder 12 includes a triple leaf spring 48.

A device 10 for obtaining a blood sample 18 of the present disclosure includes a lancet housing or lancet 14 that is removably connectable to a port 26 of a holder 12. Referring to Figs. 8, 9, and 13, in one embodiment, the lancet housing 14 includes an inlet or opening 50, an interior 52, a puncturing element 54, an engagement portion 56, a retractable mechanism 58, and a drive spring 60. In one embodiment, the puncturing element 54 is moveable between a pre-actuated position wherein the puncturing element 54 is retained within the interior 52 of the lancet housing 14 and a puncturing position wherein at least a portion of the puncturing element 54 extends through the inlet 50 of the lancet housing 14 to lance a portion of a finger 19.

Referring to Figs. 8-10, in one embodiment, the lancet housing 14 may be a separate component from the holder 12 and the collection container 16. Referring to Figs. 11-16, in some embodiments, the collection container 16 and the lancet housing 14 form a single component that is removably connectable to the port 26 of the holder 12. Referring to Figs. 17-22, in some embodiments, the collection container 16, the lancet housing 14, and the holder 12 form a single component.

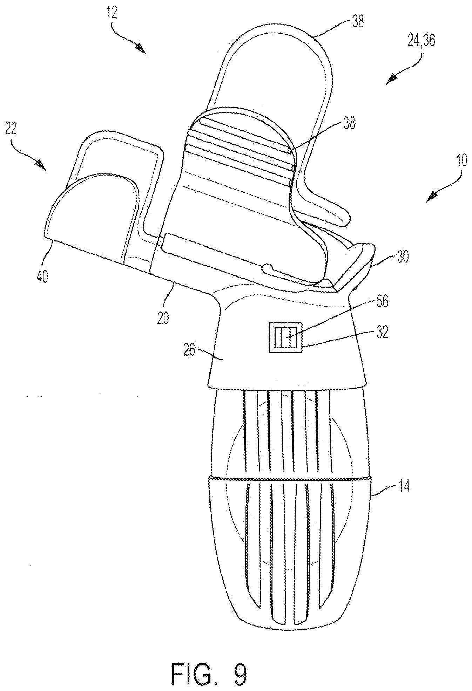

Referring to Figs. 8-10, in one embodiment, with the holder 12 and the lancet housing 14 being separate components, the lancet housing 14 is removably connectable to the port 26 of the holder 12. In such an embodiment, the lancet housing 14 includes an engagement portion 56. Referring to Fig. 9, in one embodiment, the lancet housing 14 is pushed into the port 26 of the holder 12 such that the engagement portion 56 of the lancet housing 14 is locked within the locking portion 32 of the holder 12. In this manner, the lancet housing 14 is securely connected and locked to the holder 12 such that the puncturing element 54 of the lancet housing 14 can be activated to lance or puncture a sample source, e.g., a finger 19. In some embodiments, the port 26 of the holder 12 includes a plurality of ribs for securing and locking the lancet 14 or the collection container 16 in the port 26.

To activate the lancet 14, the lancet 14 is pushed against a finger 19 to activate a retractable mechanism 58 of the lancet 14 to lance a finger 19. The lancet 14 of the present disclosure consistently delivers correct lancing depth and a pre-defined lancing location, thus ensuring a sufficient sample volume.

In one embodiment, the lancet 14 includes a drive spring 60 disposed within the interior 52 of the lancet housing 14 for biasing the puncturing element 54 toward the puncturing position. After puncturing, the puncturing element 54 is immediately retracted and safely secured within the interior 52 of the lancet housing 14.

Referring to Figs. 8-10, in one embodiment, the lancet 14 of the present disclosure is used to lance the skin of a finger 19 and then a blood sample 18 is squeezed into a collection container 16 as described in more detail below.

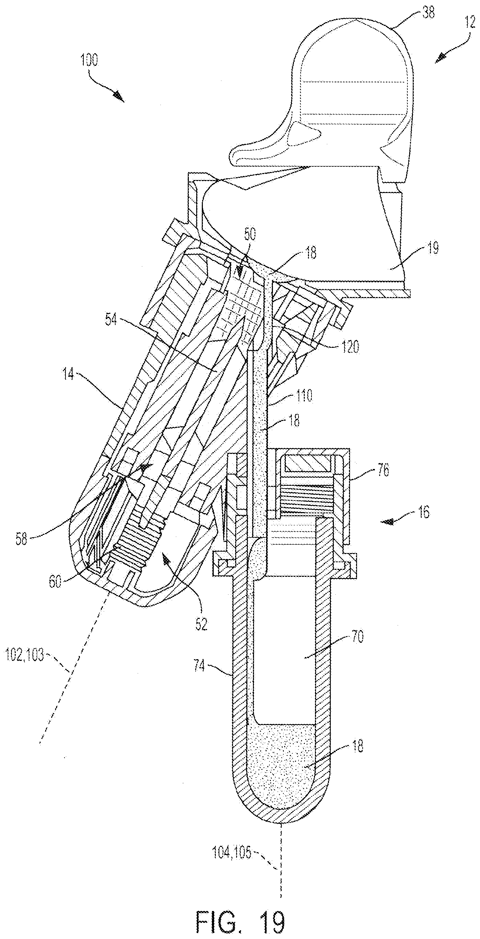

Referring to Fig. 19, in one embodiment, the lancet housing 14 of the present disclosure is used to lance the skin of a finger 19 along a lance path and then a blood sample 18 flows down a blood flow path at an angle to the lance path as described in more detail below.

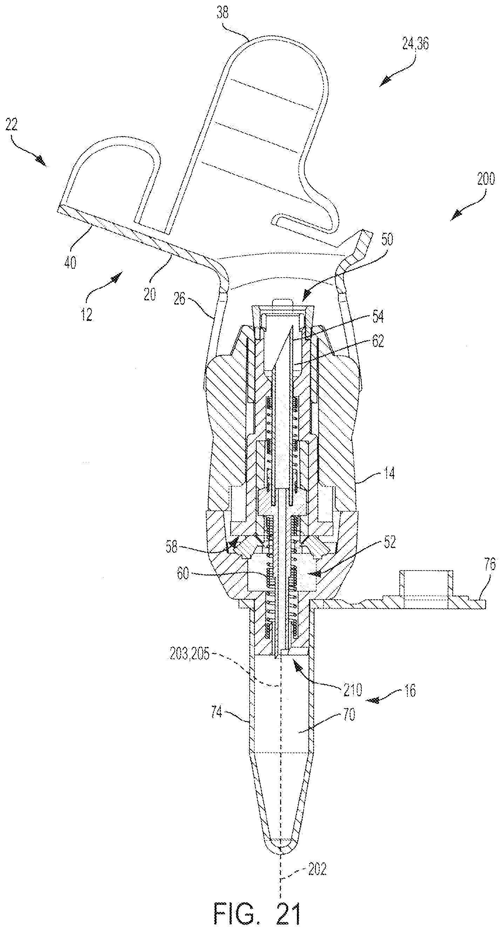

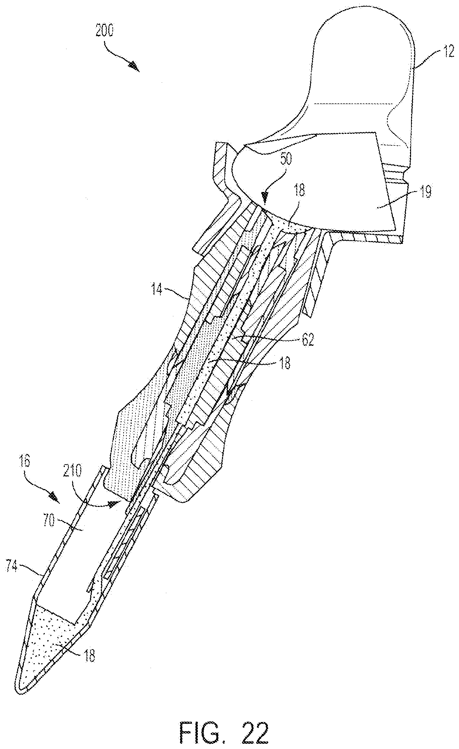

Referring to Figs. 21 and 22, in one embodiment, the lancet 14 includes a hollow needle 62. In such an embodiment, the lancet housing 14 of the present disclosure is used to lance the skin of a finger 19 along a lance path and then a blood sample 18 flows along a parallel blood flow path through the hollow needle 62 as described in more detail below.

A device 10 for obtaining a blood sample 18 of the present disclosure includes a collection container 16 that is removably connectable to the port 26 of the holder 12. The collection container 16 defines a collection cavity 70 for receiving a blood sample 18, a container engagement portion 72, a blood collector portion 74, and a cap or septum 76. Once a desired amount of blood 18 is collected within the container 16, a blood collector portion 74 is detached from the collection device 10 in order to send a collected sample 18 to a diagnostic instrument and/or testing device. The blood collector portion 74 is sealed via the cap or septum 76 once removed from the collection device 10 to protectively seal the blood sample 18 within the collection cavity 70.

Referring to Figs. 8-10, in one embodiment, the collection container 16 may be a separate component from the holder 12 and the lancet housing 14. Referring to Figs. 11-16, in some embodiments, the collection container 16 and the lancet housing 14 form a single component that is removably connectable to the port 26 of the holder 12. Referring to Figs. 17-22, in some embodiments, the collection container 16, the lancet housing 14, and the holder 12 form a single component.

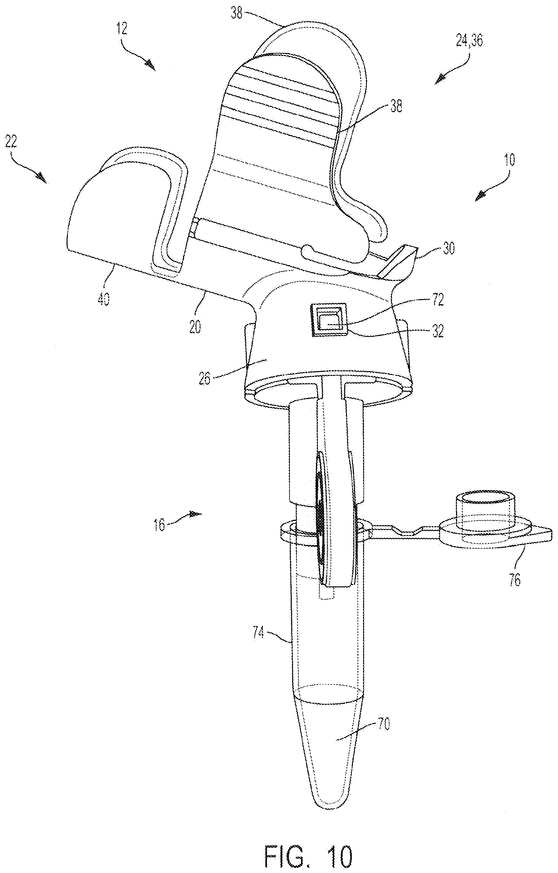

Referring to Figs. 8-10, in one embodiment, with the holder 12 and the collection container 16 being separate components, the container 16 is removably connectable to the port 26 of the holder 12. In such an embodiment, the container 16 includes a container engagement portion 72. Referring to Fig. 10, in one embodiment, the container 16 is pushed into the port 26 of the holder 12 such that the container engagement portion 72 of the container 16 is locked within the locking portion 32 of the holder 12. In this manner, the container 16 is securely connected and locked to the holder 12 such that a blood sample 18 can safely flow from the finger 19 within the holder 12 to the collection cavity 70 of the container 16.

It can be appreciated that several types of collection containers 16 can be used with the device 10 of the present disclosure. It can also be appreciated that the collection container 16 can be associated with a separate dispensing unit or the collection container 16 can include an integral dispensing portion for dispensing the blood 18 to a testing device.

Referring to Figs. 8-10 and 27-31, use of a device 10 of the present disclosure having discrete components, e.g., a holder 12, a lancet housing or lancet 14, and a collection container 16, will now be described.

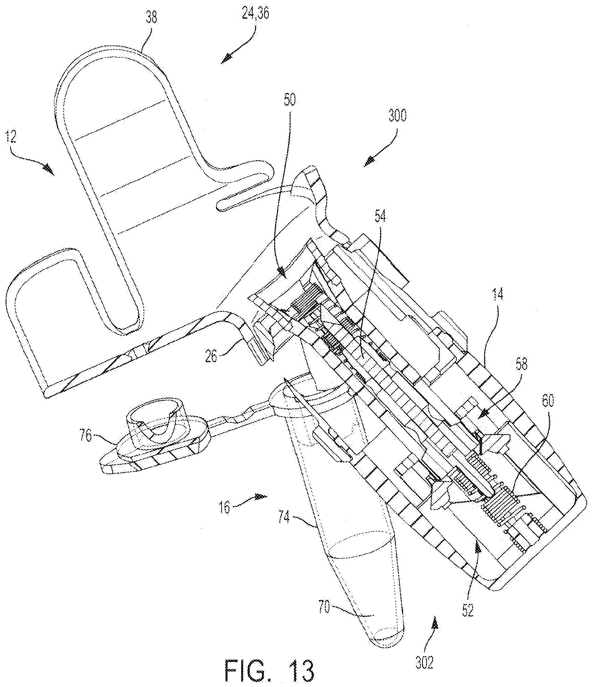

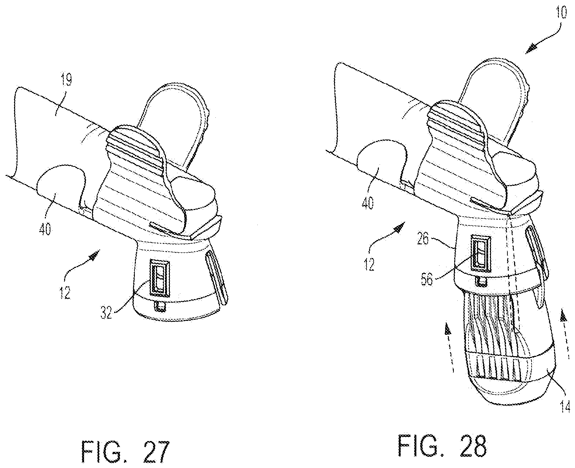

Referring to Fig. 27, first a desired finger 19 is cleaned and a holder 12 having an appropriate size for the desired finger 19 is selected and placed onto the finger 19 securely. Next, referring to Fig. 28, a lancet housing 14 is connected to the port 26 of the holder 12. As discussed above, the lancet housing 14 is pushed into the port 26 of the holder 12 such that the engagement portion 56 of the lancet housing 14 is locked within the locking portion 32 of the holder 12. In this manner, the lancet housing 14 is securely connected and locked to the holder 12 such that the puncturing element 54 (Fig. 13) of the lancet housing 14 can be activated to lance or puncture a sample source, e.g., a finger 19. With the lancet 14 connected to the port 26 of the holder 12, the lancet is in communication with the finger 19.

Referring to Fig. 28, when it is desired to activate the lancet 14 to lance the skin of a finger 19, the lancet 14 is pushed against a finger 19 to activate a retractable mechanism 58 (Fig. 13) of the lancet 14 to lance a finger 19. The lancet 14 of the present disclosure consistently delivers correct lancing depth and a pre-defined lancing location, thus ensuring a sufficient sample volume.

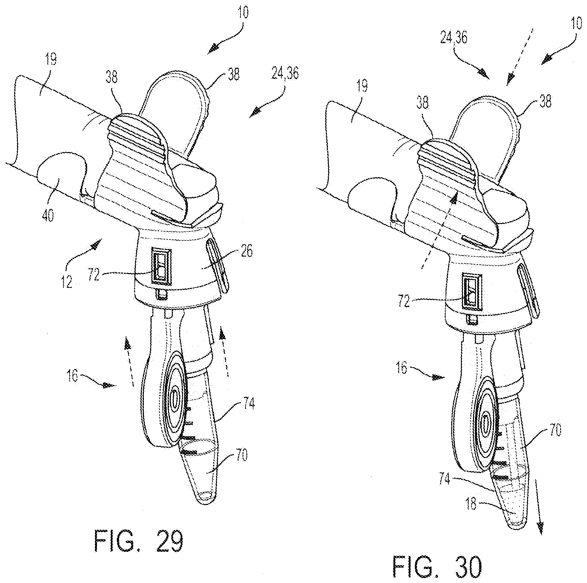

After the finger 19 is lanced to create blood 18 (Fig. 30) flow from the finger 19, the lancet 14 is removed from the holder 12 and the collection container 16 is pushed into the port 26 of the holder 12. Referring to Fig. 29, the container 16 is pushed into the port 26 of the holder 12 such that the container engagement portion 72 of the container 16 is locked within the locking portion 32 of the holder 12. In this manner, the container 16 is securely connected and locked to the holder 12 such that a blood sample 18 can safely flow from the finger 19 within the holder 12 to the collection cavity 70 of the container 16.

Referring to Figs. 29 and 30, with the container 16 properly secured to the holder 12 for collection of a blood sample 18, a user is able to repeatedly squeeze and release the wings 38 of the holder 12 to pump and/or extract blood 18 from a finger 19 until a desired amount of blood 18 is filled in a collection container 16. Advantageously, with the holder 12 placed onto a finger 19, the holder 12 does not constrict the blood flow and defines lancing and finger squeezing locations. The squeezing tabs or wings 38 provide a pre-defined range of squeezing pressure that is consistently applied throughout a finger 19. By doing so, the holder 12 provides a gentle controlled finger 19 massage that stimulates blood extraction and minimizes any potential hemolysis.

For example, referring to Figs. 2 and 3, in one embodiment, the actuation portion 24 includes a contact member 34. Referring to Fig. 2, with the actuation portion 24 in the first position, the contact member 34 is in a disengaged position, i.e., the contact member 34 is in the first position with respect to the sample source, e.g., the finger 19. Referring to Fig. 3, with the actuation portion 24 in the second position, the contact member 34 is in an engaged position, i.e., the contact member 34 is in the second position and in applied pressure contact with a sample source, e.g., the finger 19, and the actuation portion 24 of the holder 12 is able to pump and/or extract blood 18. For example, with the contact member 34 in the engaged position, the contact member 34 exerts a pressure on the sample source.

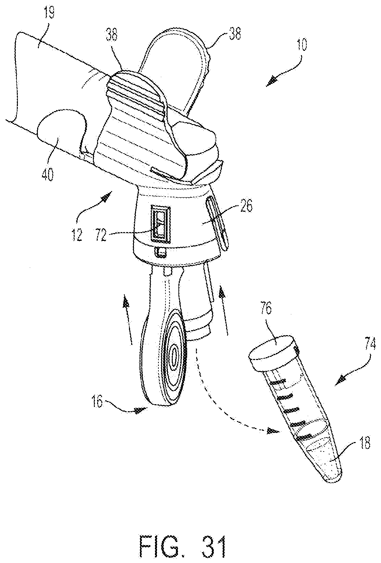

Referring to Fig. 31, once a desired amount of blood 18 is collected within the container 16, a blood collector portion 74 is detached from the collection device 10 in order to send a collected sample 18 to a diagnostic instrument and/or testing device. The blood collector portion 74 is sealed via the cap or septum 76 once removed from the collection device 10 to protectively seal the blood sample 18 within the collection cavity 70.

The devices of the present disclosure are compatible with any known testing device, whether the testing device is off-site or a point-of-care testing device. Various point-of-care testing devices are known in the art. Such point-of-care testing devices include test strips, glass slides, diagnostic cartridges, or other testing devices for testing and analysis. Test strips, glass slides, and diagnostic cartridges are point-of-care testing devices that receive a blood sample and test that blood for one or more physiological and biochemical states. There are many point-of-care devices that use cartridge based architecture to analyze very small amounts of blood bedside without the need to send the sample to a lab for analysis. This saves time in getting results over the long run, but creates a different set of challenges versus the highly routine lab environment. Examples of such testing cartridges include the i-STAT° testing cartridge from the Abbot group of companies. Testing cartridges such as the i-STAT^{®} cartridges may be used to test for a variety of conditions including the presence of chemicals and electrolytes, hematology, blood gas concentrations, coagulation, or cardiac markers. The results of tests using such cartridges are quickly provided to the clinician.

The collection container 16 may also contain a sample stabilizer, e.g., an anticoagulant, to stabilize a blood sample and/or a component of a blood sample disposed therein. The collection container 16 may also include at least one fill line(s) corresponding to a predetermined volume of sample. The collection container may also indicate/meter a collected volume of blood.

Referring to Figs. 17-19, in another exemplary embodiment, a device 100 for obtaining a blood sample 18 of the present disclosure has an at-angle flow and includes an integrated holder 12, lancet housing 14, and collection container 16. In such an embodiment, a user does not have to connect a separate lancet housing 14 to the port 26 of the holder 12, remove the lancet 14 after lancing the skin of a finger 19, and then connect a collection container 16 to the port 26 of the holder 12.

Referring to Figs. 17-19, the lancet housing 14 is permanently secured within the port 26 of the holder 12. The lancet housing 14 includes a blood flow channel 120. The collection container 16 is secured to the lancet housing 14 and includes a blood collector portion 74 that is removably connectable to a portion of the lancet housing 14.

Referring to Fig. 19, in one embodiment, with the container 16 connected to the lancet housing 14, the longitudinal axis 102 of the lancet housing 14 is at an angle to the longitudinal axis 104 of the container 16. Referring to Fig. 19, in one embodiment, the lancet housing 14 is used to lance the skin of a finger 19 along a lance path 103 and then a blood sample 18 flows down a blood flow path 105 at an angle to the lance path 103.

Referring to Figs. 17-19, in one embodiment, the device 100 includes a capillary tube 110. Referring to Fig. 19, with the container 16, i.e., the blood collector portion 74, connected to the lancet housing 14, the capillary tube 110 is in fluid communication with the inlet or opening 50 of the lancet housing 14 and the collection cavity 70 of the container 16. In one embodiment, a portion of the capillary tube 110 extends through the blood flow channel 120 of the lancet housing 14.

Referring to Figs. 17-19, the blood flow path 105 of device 100 will now be described. With a finger 19 received within the holder 12 and the puncturing element 54 in the puncturing position, the puncturing element 54 lances the finger 19 to draw a blood sample 18. For example, when it is desired to activate the lancet 14 to lance the skin of a finger 19, the lancet 14 is pushed against a finger 19 to activate a retractable mechanism 58 of the lancet 14 to lance a finger 19. The lancet 14 of the present disclosure consistently delivers correct lancing depth and a pre-defined lancing location, thus ensuring a sufficient sample volume.

The blood 18 will flow from the finger 19 to the blood flow channel 120 of the lancet housing 14. The blood 18 flows, via blood flow path 105, at an angle to the lance path 103. For example, the blood sample 18 flows through the capillary tube 110 to the collection cavity 70 of the container 16.

Referring to Figs. 20-22, in another exemplary embodiment, a device 200 for obtaining a blood sample 18 of the present disclosure has an in-line flow and includes an integrated holder 12, lancet housing 14, and collection container 16. In such an embodiment, a user does not have to connect a separate lancet housing 14 to the port 26 of the holder 12, remove the lancet 14 after lancing the skin of a finger 19, and then connect a collection container 16 to the port 26 of the holder 12.

Referring to Figs. 20-22, the lancet housing 14 is permanently secured within the port 26 of the holder 12. The lancet housing 14 includes a hollow needle 62. For example, the puncturing element 54 of the lancet 14 comprises a hollow needle 62. The collection container 16 is secured to the lancet housing 14 and includes a blood collector portion 74 that is removably connectable to a portion of the lancet housing 14.

Referring to Fig. 21, in one embodiment, with the container 16 connected to the lancet housing 14, a longitudinal axis 202 of the port 26, the lancet housing 14, and the container 16 are aligned.

Referring to Figs. 21 and 22, in one embodiment, the lancet 14 includes a hollow needle 62. In such an embodiment, the lancet housing 14 of the present disclosure is used to lance the skin of a finger 19 along a lance path 203 and then a blood sample 18 flows along a parallel blood flow path 205 through the hollow needle 62.

Referring to Figs. 20-22, in one embodiment, the lancet housing 14 includes an outlet 210. With the container 16 connected to the lancet housing 14, the outlet 210 of the lancet housing 14 is in fluid communication with the collection cavity 70 of the container 16.

Referring to Figs. 20-22, the blood flow path 205 of device 200 will now be described. With a finger 19 received within the holder 12 and the puncturing element 54 in the puncturing position, the puncturing element 54 lances the finger 19 to draw a blood sample 18. For example, when it is desired to activate the lancet 14 to lance the skin of a finger 19, the lancet 14 is pushed against a finger 19 to activate a retractable mechanism 58 of the lancet 14 to lance a finger 19. The lancet 14 of the present disclosure consistently delivers correct lancing depth and a pre-defined lancing location, thus ensuring a sufficient sample volume.

The blood 18 will flow from the finger 19 through the hollow needle 62 to the outlet 210 of the lancet housing 14 to the collection cavity 70 of the container 16. The blood 18 flows, via blood flow path 205, in line with the lance path 203.

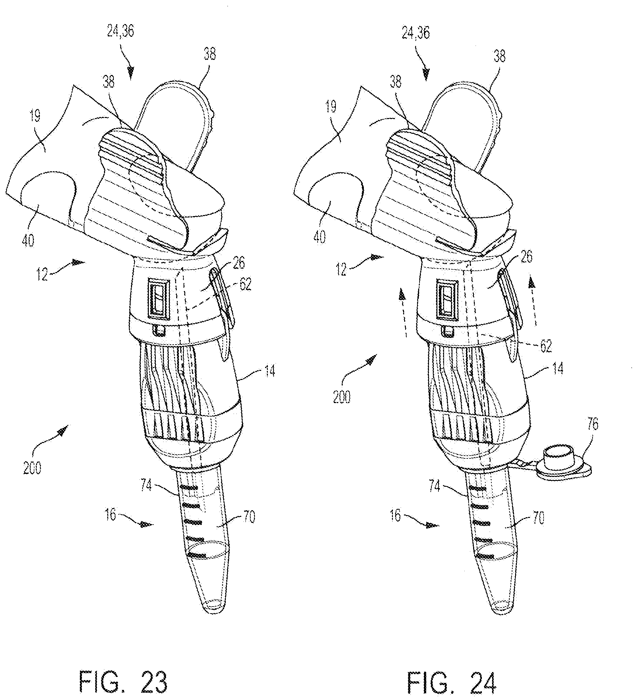

Referring to Figs. 23-26, use of a device 200 of the present disclosure having an in-line flow and including an integrated holder 12, lancet housing 14, and collection container 16, will now be described.

Referring to Fig. 23, first a desired finger 19 is cleaned and a holder 12 having an appropriate size for the desired finger 19 is selected and placed onto the finger 19 securely. In the integrated device 200 of the present disclosure, a separate lancet 14 and container 16 do not need to be selected and connected to the port 26 of the holder 12 as each of the holder 12, lancet housing 14, and collection container 16 are integrated into a single component.

Referring to Fig. 24, when it is desired to activate the lancet 14 to lance the skin of a finger 19, the lancet 14 is pushed against a finger 19 to activate a retractable mechanism 58 (Fig. 21) of the lancet 14 to lance a finger 19. The lancet 14 of the present disclosure consistently delivers correct lancing depth and a pre-defined lancing location, thus ensuring a sufficient sample volume.

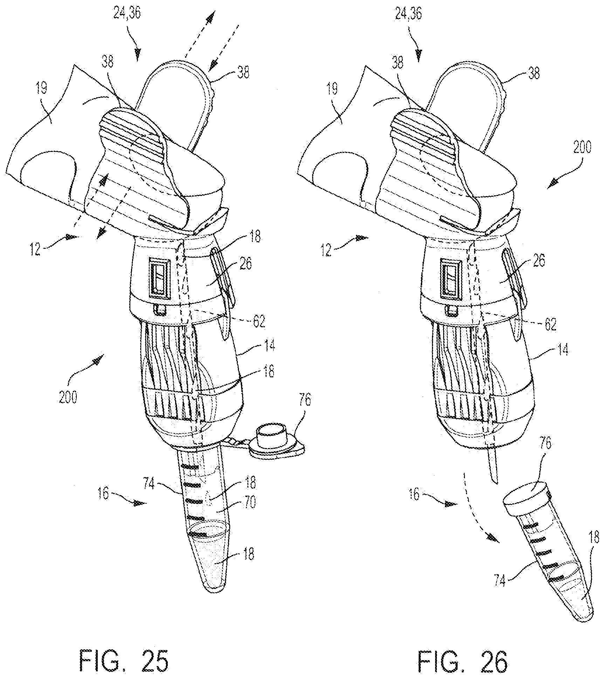

Referring to Fig. 25, after the finger 19 is lanced to create blood 18 flow from the finger 19, a user is able to repeatedly squeeze and release the wings 38 of the holder 12 to pump and/or extract blood 18 from a finger 19 until a desired amount of blood 18 is filled in a collection container 16. Advantageously, with the holder 12 placed onto a finger 19, the holder 12 does not constrict the blood flow and defines lancing and finger squeezing locations. The squeezing tabs or wings 38 provide a pre-defined range of squeezing pressure that is consistently applied throughout a finger 19. By doing so, the holder 12 provides a gentle controlled finger 19 massage that stimulates blood 18 extraction and minimizes any potential hemolysis.

Referring to Fig. 26, once a desired amount of blood 18 is collected within the container 16, a blood collector portion 74 is detached from the collection device 200 and sealed with cap or septum 76 to send a collected sample 18 to a diagnostic instrument and/or testing device. As discussed above, the collection container 16 may also contain a sample stabilizer, e.g., an anticoagulant, to stabilize blood and fill lines to indicate/meter a collected volume of blood 18.

Referring to Figs. 11-16, use of a device 300 (Figs. 11-13) having an at-angle flow and including an integrated lancet housing 14 and collection container 302 which can be connected with a separate holder 12; and a device 400 (Figs. 14-16) having an in-line flow and including an integrated lancet housing 14 and collection container 402 which can be connected with a separate holder 12 will now be described.

Referring to Figs. 11-13, the semi-integrated device 300 is used in a similar manner as the device 100 having an at-angle flow and including an integrated holder, lancet housing, and collection container described above with reference to Figs. 17-19. With use of the semi-integrated device 300, a user does not have to connect a separate lancet housing 14 to the port 26 of the holder 12, remove the lancet 14 after lancing the skin of a finger 19, and then connect a collection container 16 to the port 26 of the holder 12. In the embodiment shown in Figs. 11-13, a user only needs to connect an integrated lancet housing and collection container component 302 to the port 26 of the holder 12.

Referring to Figs. 14-16, the semi-integrated device 400 is used in a similar manner as the device 200 having an in-line flow and including an integrated holder, lancet housing, and collection container described above with reference to Figs. 20-22. With use of the semi-integrated device 400, a user does not have to connect a separate lancet housing 14 to the port 26 of the holder 12, remove the lancet 14 after lancing the skin of a finger 19, and then connect a collection container 16 to the port 26 of the holder 12. In the embodiment shown in Figs. 14-16, a user only needs to connect an integrated lancet housing and collection container component 402 to the port 26 of the holder 12.

Any of the devices for obtaining a blood sample of the present disclosure can be used as a self-standing disposable device and/or in association with an external power source for pain reduction control. For example, a portion of holder 12 may include embedded electrodes which receive a signal from an external pain control module to deliver at least one of heat, vibration, or transcutaneous electrical nerve stimulation (TENS) for pain reduction control. The devices for obtaining a blood sample of the present disclosure may also include various options for on-board plasma separation. The devices for obtaining a blood sample of the present disclosure may also include a unique sample identifier that can be paired with patient information at the time of collection. The devices for obtaining a blood sample of the present disclosure may also include on-board diagnostic feedback at the time of collection. A device for obtaining a blood sample of the present disclosure may also allow for dual collection, e.g., the collection of two samples into two separate containers, using multiple collection ports which enable the collection of multiple samples from the same source and treating the samples with different sample stabilizers, such as anticoagulants.

A device for obtaining a blood sample of the present disclosure significantly simplifies and de-skills large volume capillary collection from a finger relative to the conventional capillary collection using lancet and capillary tube. The devices of the present disclosure eliminate blood exposure and prevents device reuse.

The devices for obtaining a blood sample of the present disclosure simplify, deskill, and streamline the collection process. This is all achieved by a self-contained closed system device which after it is placed onto a finger will provide lancing, blood extraction, stabilization and containment functions, all in one unit.

The devices for obtaining a blood sample of the present disclosure may be associated with a self-standing unit that provides automated pumping, controlled finger squeezing, and automated sample labeling and processing.

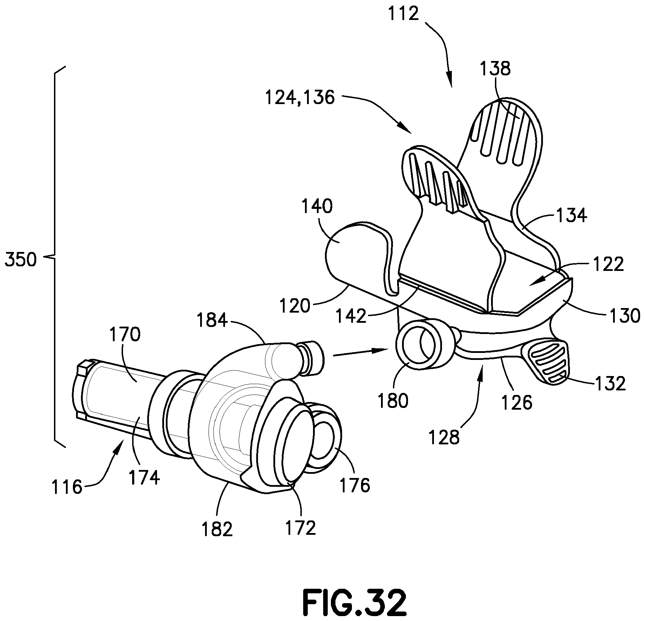

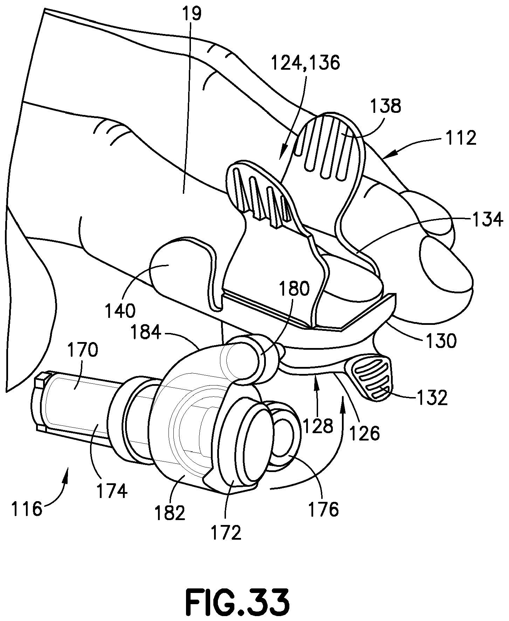

Referring to Figs. 32-37d, exemplary embodiments of holders 112 of the present disclosure are described that are able to receive a sample source, e.g., a finger 19, for supplying a biological sample, such as a blood sample. A holder 112 of the present disclosure generally includes a finger receiving portion 120 having a first opening 122, an actuation portion 124, a port 126 having a second opening 128, and a finger end guard 130. In one embodiment, the finger end guard 130 provides a stop portion for properly aligning and securing a finger 19 within the holder 112.

The first opening 122 of the finger receiving portion 120 is configured for receiving a sample source, e.g., a finger 19, for supplying a biological sample, such as a blood sample. It can be appreciated that the sample source could include other parts of the body capable of fitting within the first opening 122. The port 126 is in communication with the finger receiving portion 120. For example, with a finger 19 received within the holder 112, the port 126 is in communication with a portion of the finger 19. A holder 112 of the present disclosure can be sized to accommodate all finger sizes.

The second opening 128 of the port 126 is configured for receiving a lancet housing 14 and a collection container 116 as described in more detail below. In one embodiment, the port 126 includes a locking portion 132 for securely receiving the lancet housing 14 and the collection container 116 within the port 126.

In one embodiment, the actuation portion 124 is transitionable between a first position in which the holder 112 defines a first diameter and a second position in which the holder 112 defines a second diameter, wherein the second diameter is less than the first diameter. In one embodiment, the actuation portion 124 is transitionable between a first position in which the holder 112 defines a first elliptical shape, and a second position in which the holder 112 defines a second elliptical shape, wherein the first elliptical shape is different than the second elliptical shape. In this manner, with the holder 112 in the second position with a reduced diameter, a portion of the holder 112 contacts the sample source and the actuation portion 124 of the holder 112 is able to pump and/or extract blood as described in more detail below.

In one embodiment, the actuation portion 124 includes a contact member 134. With the actuation portion 124 in the first position, the contact member 134 is in a disengaged position, i.e., the contact member 134 is provided in a first position with respect to a sample source, e.g., the finger 19, such that the contact member 134 may be in slight contact therewith. With the actuation portion 124 in the second position, the contact member 134 is in an engaged position, i.e., the contact member 134 is provided in a second position with respect to the sample source, e.g., the finger 19, such that the contact member 134 is in an applied pressure contact with the finer 19, and the actuation portion 124 of the holder 112 is able to pump and/or extract blood. For example, with the contact member 134 in the engaged position, the contact member 134 exerts a pressure on the sample source.

In one embodiment, the actuation portion 124 includes a pumping member 136 for applying pressure to the sample source, e.g., the finger 19. In one embodiment, the pumping member 136 comprises a pair of opposed tabs or wings 138. In such an embodiment, each tab 138 may include a contact member 134. In one embodiment, the holder 112 includes a living hinge portion 142. The living hinge portion 142 allows a user to squeeze the wings 138 between a first position and a second position.

Advantageously, the holder 112 of the present disclosure allows a user to repeatedly squeeze and release the wings 138 to pump and/or extract blood from a finger 19 until a desired amount of blood is filled in a collection container 116.

Advantageously, with the holder 112 placed onto a finger 19, the holder 112 does not constrict the blood flow and defines lancing and finger squeezing locations. The squeezing tabs or wings 138 provide a pre-defined range of squeezing pressure that is consistently applied throughout a finger 19. By doing so, the holder 112 provides a gentle controlled finger massage that stimulates blood extraction and minimizes any potential hemolysis.

In one embodiment, the holder 112 includes a stability extension portion 140. This provides additional support for the holder 112 to be securely placed onto a finger 19. In one embodiment, the finger receiving portion 120 forms a generally C-shaped member and includes a plurality of inner gripping members for providing additional grip and support for the holder 112 to be securely placed onto a finger 19.

In one embodiment, the finger receiving portion 120 is formed of a flexible material. In some embodiments, the finger receiving portion 120 and the port 126 are formed from a flexible material.

A device 350 for obtaining a blood sample of the present disclosure includes a lancet housing or lancet 114 that is removably connectable to a port 126 of a holder 112. In one embodiment, the lancet 114 includes an inlet or opening, an interior, a puncturing element, an engagement portion, a retractable mechanism, and a drive spring, similar to the lancet 14 described hereinabove. In one embodiment, the puncturing element is moveable between a pre-actuated position wherein the puncturing element is retained within the interior of the lancet 114 and a puncturing position wherein at least a portion of the puncturing element extends through the inlet of the lancet 114 to lance a portion of a finger 19.

In one embodiment, the lancet 114 may be a separate component from the holder 112 and the collection container 116. In some embodiments, the collection container 116 and the lancet 114 form a single component that is removably connectable to the port 126 of the holder 112. In some embodiments, the collection container 116, the lancet 114, and the holder 112 form a single component.

In one embodiment, with the holder 112 and the lancet 114 being separate components, the lancet 114 is removably connectable to the port 126 of the holder 112. In such an embodiment, the lancet 114 includes an engagement portion. In one embodiment, the lancet 114 is pushed into the port 126 of the holder 112 such that the engagement portion of the lancet 114 is locked within the port 126 of the holder 112. In this manner, the lancet 114 is securely connected and locked to the holder 112 such that the puncturing element of the lancet 114 can be activated to lance or puncture a sample source, e.g., a finger 19. In some embodiments, the port 126 of the holder 112 includes a plurality of ribs for securing and locking the lancet 114 or the collection container 116 in the port 126.

To activate the lancet 114, the lancet 114 is pushed against a finger 19 to activate a retractable mechanism of the lancet 114 to lance a finger 19. The lancet 114 of the present disclosure consistently delivers correct lancing depth and a pre-defined lancing location, thus ensuring a sufficient sample volume.

In one embodiment, the lancet 114 includes a drive spring disposed within the interior of the lancet 114 for biasing the puncturing element toward the puncturing position. After puncturing, the puncturing element is immediately retracted and safely secured within the interior of the lancet 114.

In one embodiment, the lancet 114 of the present disclosure is used to lance the skin of a finger 19 and then a blood sample is squeezed into a collection container 116 as described in more detail below.

In one embodiment, the lancet 114 of the present disclosure is used to lance the skin of a finger 19 along a lance path and then a blood sample flows down a blood flow path at an angle to the lance path as described in more detail below.

In one embodiment, the lancet 114 includes a hollow needle. In such an embodiment, the lancet 114 of the present disclosure is used to lance the skin of a finger 19 along a lance path and then a blood sample flows along a parallel blood flow path through the hollow needle as described in more detail below.

The device 350 for obtaining a blood sample of the present disclosure includes a collection container 116 that is removably connectable to the port 126 of the holder 112. The collection container 116 defines a collection cavity 170 for receiving a blood sample, a blood collector portion 174, and a cap or septum 176. Once a desired amount of blood is collected within the container 116, a blood collector portion 174 is detached from the collection device 350 in order to send a collected sample to a diagnostic instrument and/or testing device. The blood collector portion 174 is sealed via the cap or septum 176 once removed from the device 350 to protectively seal the blood sample within the collection cavity 170.

In one embodiment, with the holder 112 and the collection container 116 being separate components, a container engagement portion 182 that holds the collection container 116 may be removably or fixedly connectable to a connection interface 180 of the holder 112. The container engagement portion 182 may define an opening to receive and hold the collection container 116. The collection container 116 may be held in the container engagement portion 182 via any suitable means, including a friction fit, a mechanical fit, or any alternative removable connection to permit the collection container 116 to be separated from the container engagement portion 182 after the blood sample has been collected in the collection cavity 170. The container engagement portion 182 may also include a container locking portion 172 that is configured to engage with the port 126 of the holder 112 to lock the container engagement portion 182 within the port 126. Referring to Fig. 32, in one embodiment, the container engagement portion 182 includes a protrusion 184 that is pushed or directed into the connection interface 180 of the holder 112 such that the container engagement portion 182 of the container 116 is locked within the connection interface 180 of the holder 112. In this manner, the container 116 is securely connected and locked to the holder 112 such that a blood sample can safely flow from the finger 19 within the holder 112 to the collection cavity 170 of the container 116. The connection interface 180 and the container engagement portion 182 may be connected prior to lancing or at a manufacturing location. This pre-attached pivot mechanism offers a quick and connected connection of the collection container 116 to the holder 112. Due to the connection between the connection interface 180 of the holder 112 and the container engagement portion 182, the container engagement portion 182 is rotatably movable relative to the holder 112. Therefore, with the collection container 116 held in the container engagement portion 182, the collection container 116 can be rotatably moved between a first position in which the collection container 116 is positioned away from the port 126 on the holder 112 and a second position in which the collection container 116 is positioned in-line with the port 126 of the holder 112 to collect a blood sample from a patient's finger 19.

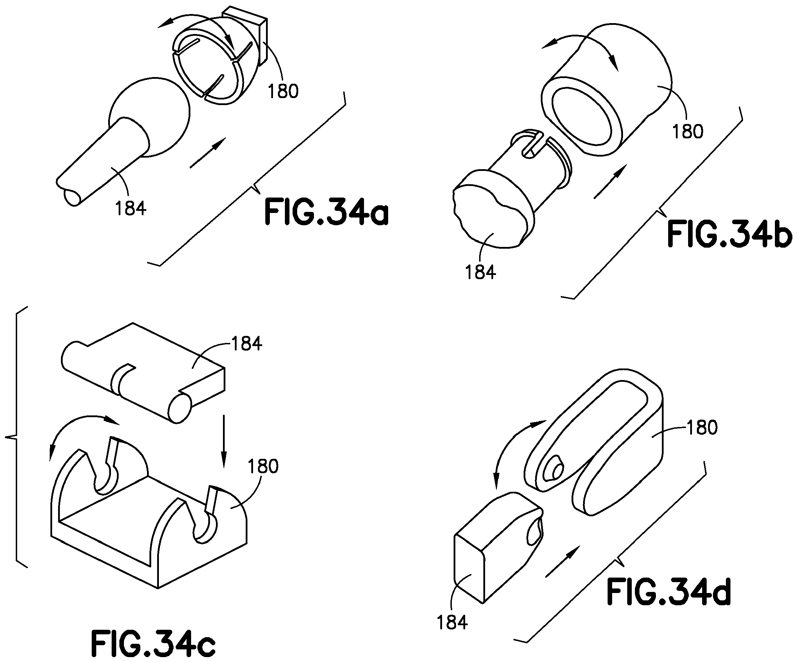

With reference to Figs. 34a-34d, alternative connection arrangements between the connection interface 180 and the protrusion 184 are illustrated and described. As shown in Fig. 34a, the connection interface 180 and the protrusion 184 may include a ball joint connection. It is contemplated that either the connection interface 180 or the protrusion 184 may include a ball and the other of the connection interface 180 and the protrusion 184 may include a socket to receive the ball. As shown in Fig. 34b, the connection interface 180 and the protrusion 184 may include a peg and hole pivot joint connection. It is contemplated that either the connection interface 180 or the protrusion 184 may include a peg and the other of the connection interface 180 and the protrusion 184 may include a hole or socket to receive the peg. With reference to Fig. 34c, the connection interface 180 and the protrusion 184 may include a pin and C-clip pivot joint connection. It is contemplated that either the connection interface 180 or the protrusion 184 may include a pin and the other of the connection interface 180 and the protrusion 184 may include a C-clip to receive the pin. With reference to Fig. 34d, the connection interface 180 and the protrusion 184 may include a pin and pocket pivot joint connection. It is contemplated that either the connection interface 180 or the protrusion 184 may include a pin and the other of the connection interface 180 and the protrusion 184 may include a pocket to receive the pin. It is further contemplated herein that other alternative connection arrangements between the connection interface 180 and the protrusion 184 may also be employed in the present invention. The previously described connections are non-limiting.

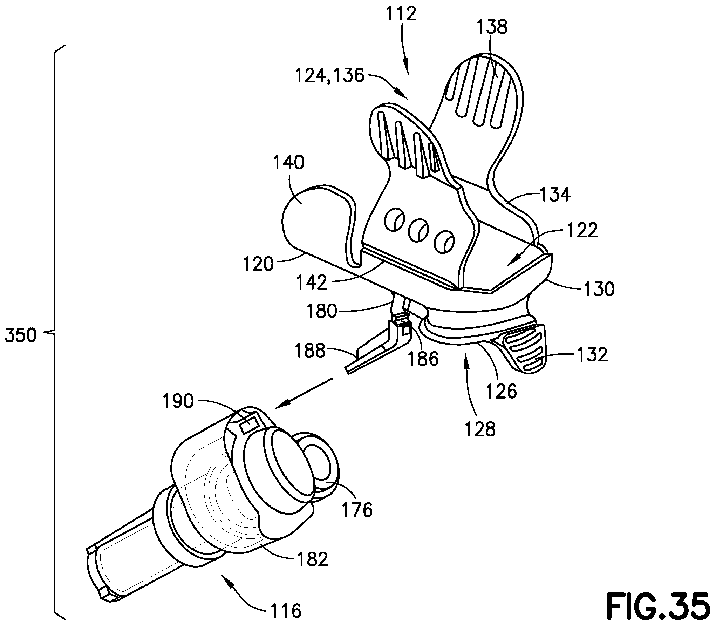

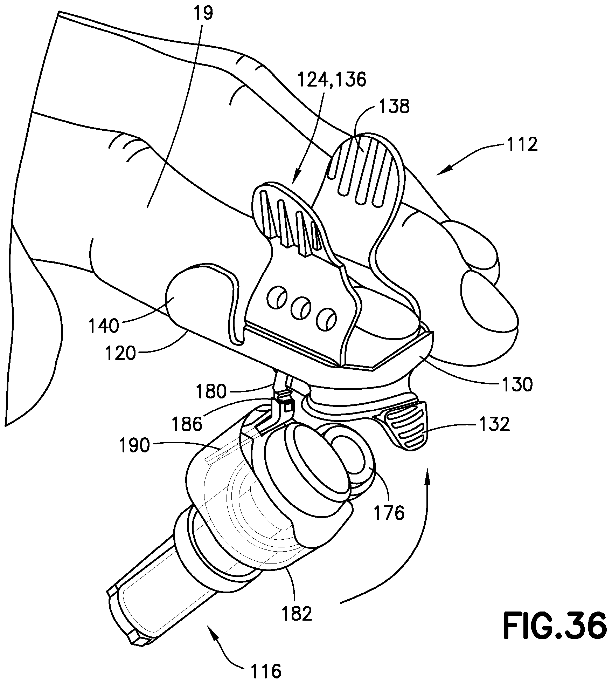

Referring to Figs. 35 and 36, in one embodiment, the connection interface 180 of the holder 112 may include a living hinge 186 and a connection tab 188 that is receivable in an aperture 190 defined in the container engagement portion 182. In this manner, the container 116 is securely connected and locked to the holder 112 such that a blood sample can safely flow from the finger 19 within the holder 112 to the collection cavity 170 of the collection container 116. The connection interface 180 and the container engagement portion 182 may be connected prior to lancing or at a manufacturing location. This pre-attached pivot mechanism offers a quick and connected connection of the collection container 116 to the holder 112. Due to the living hinge 186 provided with the connection interface 180 of the holder 112, the container engagement portion 182 is rotatably movable relative to the holder 112. Therefore, with the collection container 116 held in the container engagement portion 182, the collection container 116 can be rotatably moved between a first position in which the collection container 116 is positioned away from the port 126 on the holder 112 and a second position in which the collection container 116 is positioned in-line with the port 126 of the holder 112 to collect a blood sample from a patient's finger 19.

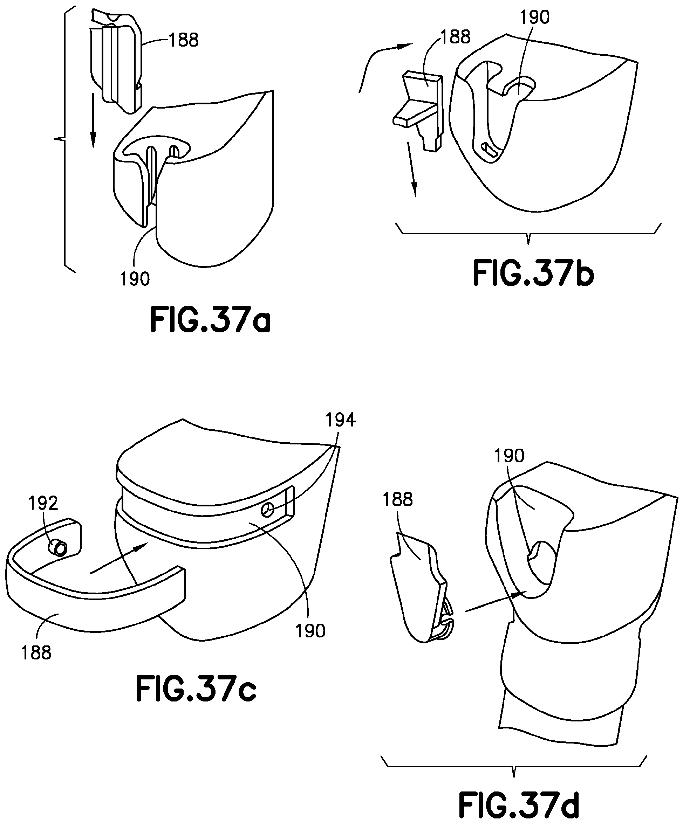

With reference to Figs. 37a-37d, alternative connection arrangements between the connection tab 188 and the aperture 190 are illustrated and described. As shown in Fig. 37a, the connection tab 188 and the aperture 190 may include a keyed snap fit connection. It is contemplated that the connection tab 188 may be keyed to be received within the aperture 190 that has a corresponding shape to the key of the connection tab 188. The connection tab 188 may be snap fit into the aperture 190. As shown in Fig. 37b, the connection tab 188 and the aperture 190 may include a hook and snap connection. It is contemplated that the connection tab 188 may be a hook element that is to be received within the aperture 190 in a rotatable manner. The connection tab 188 may be snap fit into the aperture 190. A first end of the hook element of the connection tab 188 may be snapped into the aperture 190 and then a second end of the hook element of the connection tab 188 may be rotated in an opposite direction to snap into an opposing end of the aperture 190. As shown in Fig. 37c, the connection tab 188 and the aperture 190 may include a C-clip and snap connection. It is contemplated that the connection tab 188 may be a C-clip that is to be received within the aperture 190 with a snap fit. The C-clip of the protrusion tab 188 may include inner extensions 192 that are snap fit into corresponding holes 194 defined in the aperture 190. As shown in Fig. 37d, the connection tab 188 and the aperture 190 may include a peg and profiled engagement connection. It is contemplated that the connection tab 188 may be a peg that is to be received within the aperture 190 that has a corresponding profile to the peg. The connection tab 188 may be snap fit into the aperture 190. It is further contemplated herein that other alternative connection arrangements between the connection tab 188 and the aperture 190 may also be employed in the present invention. The previously described connections are non-limiting.

Referring to Fig. 32, in one embodiment, the container 116 is pushed into the port 126 of the holder 112 such that the container locking portion 172 of the container engagement portion 182 is locked within the locking portion 132 of the holder 112. In this manner, the collection container 116 is securely connected and locked to the holder 112 such that a blood sample can safely flow from the finger 19 within the holder 112 to the collection cavity 170 of the collection container 116.

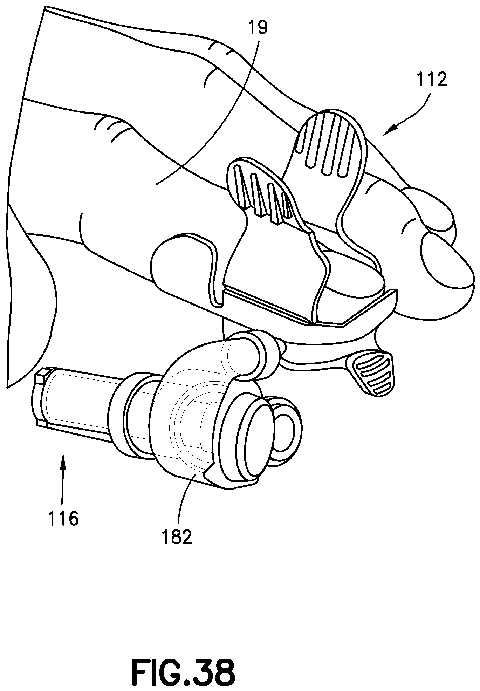

Referring to Figs. 38-42, use of a device 350 of the present disclosure having an in-line flow and including a holder 112, lancet 114, and collection container 116, will now be described.

Referring to Fig. 38, first a desired finger 19 is cleaned and a holder 112 having an appropriate size for the desired finger 19 is selected and placed onto the finger 19 securely.

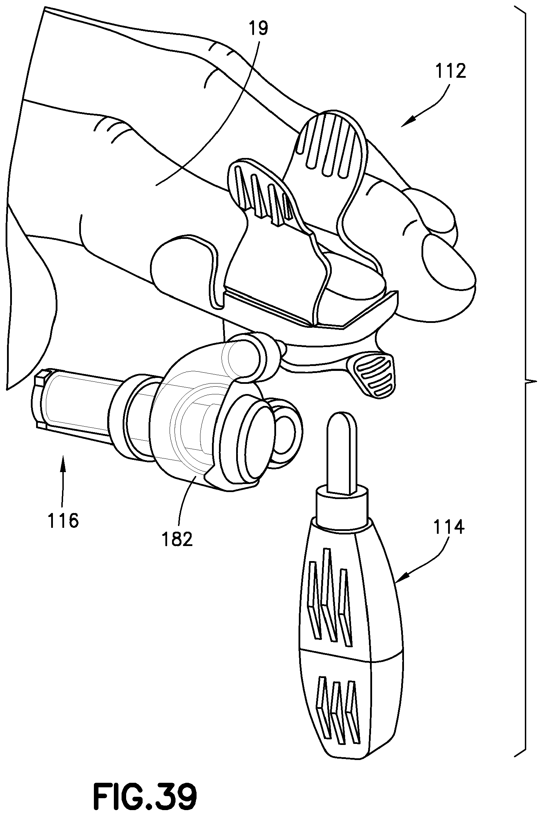

Referring to Fig. 39, when it is desired to activate the lancet 114 to lance the skin of a finger 19, the lancet 114 is pushed against a finger 19 to activate a retractable mechanism of the lancet 114 to lance a finger 19. The lancet 114 of the present disclosure consistently delivers correct lancing depth and a pre-defined lancing location, thus ensuring a sufficient sample volume.

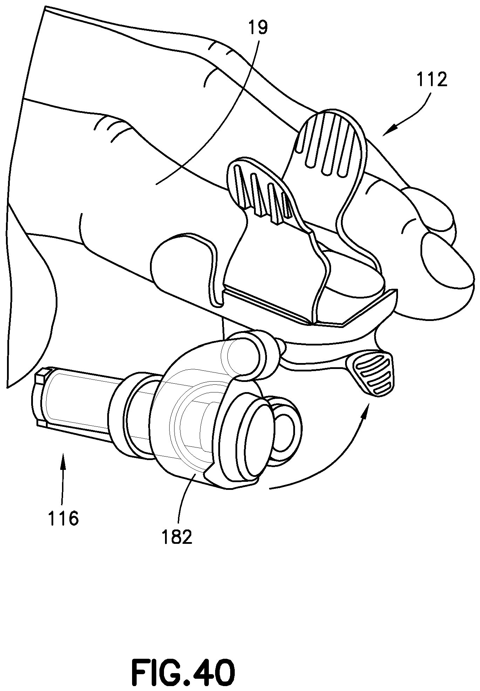

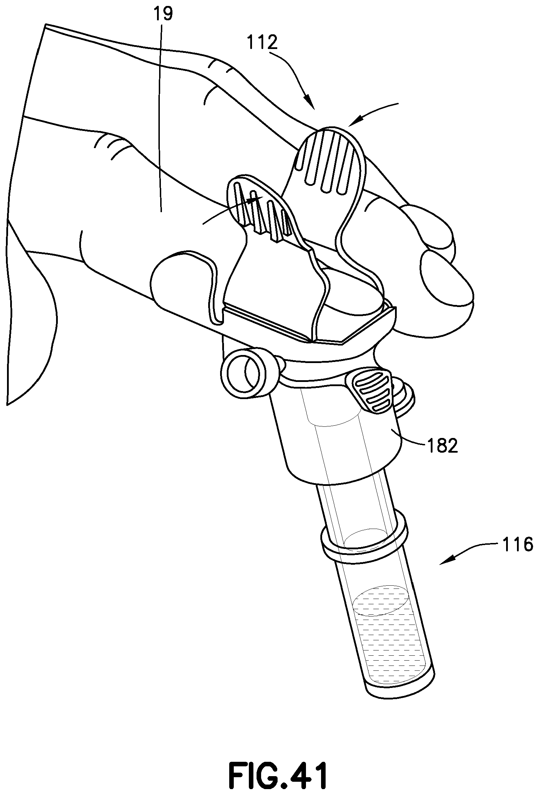

Referring to Fig. 40, after the finger 19 is lanced to create blood flow from the finger 19, a user can rotation the collection container 116 into the second position to lock into the holder 112. Referring to Fig. 41, once the container engagement portion 182 has been locked to the holder 112, a user is able to repeatedly squeeze and release the wings 138 of the holder 112 to pump and/or extract blood from a finger 19 until a desired amount of blood is filled in a collection container 116. Advantageously, with the holder 112 placed onto a finger 19, the holder 112 does not constrict the blood flow and defines lancing and finger squeezing locations. The squeezing tabs or wings 138 provide a pre-defined range of squeezing pressure that is consistently applied throughout a finger 19. By doing so, the holder 112 provides a gentle controlled finger 19 massage that stimulates blood extraction and minimizes any potential hemolysis.

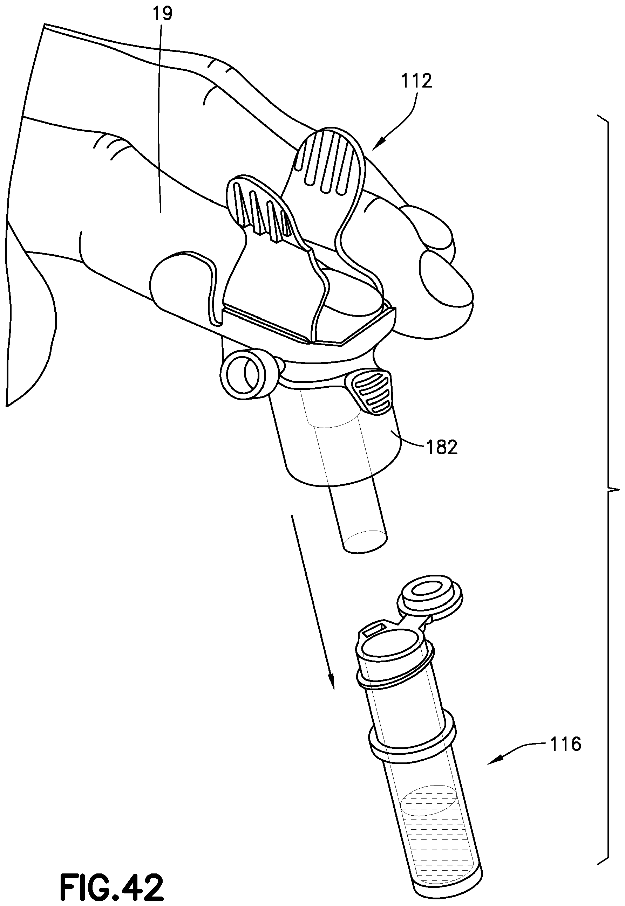

Referring to Fig. 42, once a desired amount of blood is collected within the container 116, the collection container 116 is detached from the container engagement portion 182 and sealed with cap or septum 176 to send a collected sample to a diagnostic instrument and/or testing device. As discussed above, the collection container 116 may also contain a sample stabilizer, e.g., an anticoagulant, to stabilize blood and fill lines to indicate/meter a collected volume of blood. In the event additional blood is to be collected from the patient, a second collection container 116 can be attached to the container engagement portion 182 and the holder 112 can be used again to pump blood into the second collection container 16.

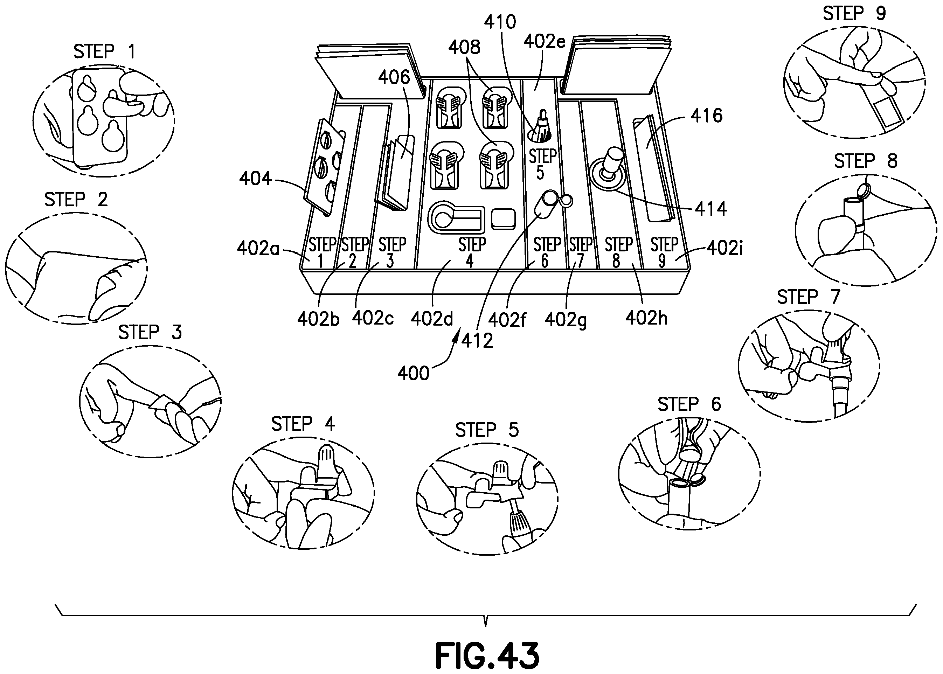

With reference to Fig. 43, a tray 400 for holding a plurality of different holders 112 that can be sized and used on a patient's finger 19 is described. The tray 400 may also include separate sections 402a-402i that hold individual components that can assist a user in using the holder 112 to collection a patient's blood sample in a collection container 116. Each section 402a-401i may include a label indicating which step of the process each section 402a-402i corresponds to indicate to a user the specific step in the process that each section 402a-402i applies to. The tray 400 may hold all of the necessary components that a user would need to pull a blood sample from a patient's finger 19. The tray 400 is designed to guide a user to perform the workflow in a correct sequence in a timely fashion. In the first section 402a, labelled "Step 1", a finger measuring card 404 is held in a slot defined in the tray 400. The finger measuring card 404 may include several different apertures that have different diameters corresponding to different sized holders 112. To ensure the proper holder 112 is used on the patient, the patient will insert his/her finger 19 in the different apertures in the finger measuring card 404 to determine the specific holder 112 that corresponds to the finger's finger 19.

In the second section 402b, labelled "Step 2", an instruction is provided to indicate to the user that the patient's hand/finger should be warmed. In the third section 402c, labelled "Step 3", a slot is defined to hold a plurality of disinfecting wipes 406. The user can use a disinfecting wipe 406 to disinfect the puncture site on the patient's finger 19. In the fourth section 402d, labelled "Step 4", a plurality of apertures 408 are defined in the section 402c of the tray 400 to holder the different sized holders 112. According to the measurement of the patient's finger 19 using the finger measuring card 404, the user will pick out the appropriate holder 112 that is sized for the patient's finger 19. After the proper holder 112 is identified, the user will insert the patient's finger 19 into the holder 112, as described above.

In the fifth section 402e, labelled "Step 5", an aperture 410 is defined in the tray 400 to hold a lancet 114. The user can remove the lancet 114 from the tray and use the lancet 114 to puncture the patient's finger 19, as described above. In the sixth section 402f, labelled "Step 6", an aperture 412 is defined in the tray 400 to hold a collection container 116. The collection container 116 can be removed from the tray 400 and attached to a container engagement portion 182 connected to the holder 112 to collect a blood sample from the patient's finger 19, as described above.

In the seventh section 402g, labelled "Step 7", an instruction to pump the patient's finger 19 using the holder 112 is provided. In the eighth section 402h, labelled "Step 8", an aperture 414 is defined in the tray 400 to hold the collection container 116 after the blood sample has been pulled from the patient's finger 19. In the ninth section 402i, labelled "Step 9", an aperture 416 is defined in the tray 400 to hold bandages 418 that can be applied to the patient's finger 19 after the holder 112 has been removed therefrom.

## Claims

1. A device for obtaining a blood sample (18), the device comprising:
a holder (12) for receiving a sample source, the holder (12) having an actuation portion (24) and a port (26);
a container engagement portion (56) connected to the holder (12); and
a collection container (16) removably connectable to the container engagement portion (56);
**characterized by** the container engagement portion (56) allows the collection container (16) to rotate between a first position in which the collection container (16) is spaced from the port (26) and a second position in which the collection container (16) is in fluid communication with the port (26).

2. The device of claim 1, **characterized by** the actuation portion (24) is transitionable between a first position in which the holder (12) defines a first ellipse and a second position in which the holder (12) defines a second ellipse, wherein the second ellipse is different than the first ellipse.

3. The device of claim 1, **characterized by** the actuation portion (24) includes a contact member (34).

4. The device of claim 3, **characterized by** the actuation portion (24) is transitionable between a first position in which the contact member (34) is in a disengaged position and a second position in which the contact member (34) is in an engaged position.

5. The device of claim 4, **characterized by**, with the contact member (34) in the engaged position, the contact member (34) exerts a pressure on the sample source.

6. The device of claim 1, **characterized by** the actuation portion (24) includes a pumping member (36) for applying pressure to the sample source.

7. The device of claim 6, **characterized by** the pumping member (36) comprises a pair of opposed tabs (38).

8. The device of claim 1, **characterized by** the sample source is a finger (19).

9. The device of claim 8, **characterized by**, with the finger (19) received within the holder (12), the port (26) is in communication with a portion of the finger (19).

10. The device of claim 1, **characterized by** the container engagement portion (56) and the holder (12) are connected via a ball joint, a peg and hole pivot joint, a pin and C-clip pivot j oint, or a pin and pocket pivot joint.

11. The device of claim 1, **characterized by** the holder (12) comprises a living hinge (186) that is connectable to the container engagement portion (56).

12. The device of claim 11, **characterized by** the living hinge (186) is connected to the container engagement portion (56) via a keyed snap-fit arrangement, a hook and snap-fit arrangement, a C-clip and snap-fit arrangement, or a peg and profiled engagement.

13. The device of claim 1, **characterized by** the container defining a collection cavity (70).

## Patentansprüche

1. Vorrichtung zum Gewinnen einer Blutprobe (18), wobei die Vorrichtung umfasst:
einen Halter (12) zum Aufnehmen einer Probenquelle, wobei der Halter (12) einen Betätigungsabschnitt (24) und einen Anschluss (26) aufweist;
einen Behältereinrückabschnitt (56), der mit dem Halter (12) verbunden ist; und
einen Sammelbehälter (16), der abnehmbar mit dem Behältereinrückabschnitt (56) verbunden werden kann;
**dadurch gekennzeichnet, dass** der Behältereinrückabschnitt (56) es dem Sammelbehälter (16) ermöglicht, zwischen einer ersten Stellung, in der der Sammelbehälter (16) vom Anschluss (26) beabstandet ist, und einer zweiten Stellung, in der der Sammelbehälter (16) mit dem Anschluss (26) in Fluidkommunikation steht, zu drehen.

2. Vorrichtung nach Anspruch 1, **dadurch gekennzeichnet, dass** der Betätigungsabschnitt (24) zwischen einer ersten Stellung, in der der Halter (12) eine erste Ellipse definiert, und einer zweiten Stellung, in der der Halter (12) eine zweite Ellipse definiert, überführt werden kann, wobei sich die zweite Ellipse von der ersten Ellipse unterscheidet.

3. Vorrichtung nach Anspruch 1, **dadurch gekennzeichnet, dass** der Betätigungsabschnitt (24) ein Kontaktglied (34) beinhaltet.

4. Vorrichtung nach Anspruch 3, **dadurch gekennzeichnet, dass** der Betätigungsabschnitt (24) zwischen einer ersten Stellung, in der sich das Kontaktglied (34) in einer ausgerückten Stellung befindet, und einer zweiten Stellung, in der sich das Kontaktglied (34) in einer eingerückten Stellung befindet, überführt werden kann.

5. Vorrichtung nach Anspruch 4, **dadurch gekennzeichnet, dass**, wenn sich das Kontaktglied (34) in der eingerückten Stellung befindet, das Kontaktglied (34) einen Druck auf die Probenquelle ausübt.

6. Vorrichtung nach Anspruch 1, **dadurch gekennzeichnet, dass** der Betätigungsabschnitt (24) ein Pumpglied (36) zum Anlegen von Druck auf die Probenquelle beinhaltet.

7. Vorrichtung nach Anspruch 6, **dadurch gekennzeichnet, dass** das Pumpglied (36) ein Paar gegenüberliegender Laschen (38) umfasst.

8. Vorrichtung nach Anspruch 1, **dadurch gekennzeichnet, dass** es sich bei der Probenquelle um einen Finger (19) handelt.

9. Vorrichtung nach Anspruch 8, **dadurch gekennzeichnet, dass** wenn der Finger (19) im Halter (12) aufgenommen ist, der Anschluss (26) mit einem Abschnitt des Fingers (19) in Kommunikation steht.

10. Vorrichtung nach Anspruch 1, **dadurch gekennzeichnet, dass** der Behältereinrückabschnitt (56) und der Halter (12) über ein Kugelgelenk, ein Zapfen-/Loch-Schwenkgelenk, ein Stift-/C-Clip-Schwenkgelenk, oder ein Stift/Tasche-Schwenkgelenk verbunden sind.

11. Vorrichtung nach Anspruch 1, **dadurch gekennzeichnet, dass** der Halter (12) ein bewegliches Scharnier (186) umfasst, das mit dem Behältereinrückabschnitt (56) verbunden werden kann.

12. Vorrichtung nach Anspruch 11, **dadurch gekennzeichnet, dass** das bewegliche Scharnier (186) über eine Einrast-Anordnung mit Taste, eine Haken-/Einrast-Anordnung, eine C-Clip-/Einrast-Anordnung, oder einen Zapfen und profilierte Einrückung mit dem Behältereinrückabschnitt (56) verbunden ist.

13. Vorrichtung nach Anspruch 1, **dadurch gekennzeichnet, dass** der Behälter eine Sammelkammer (70) definiert.

## Revendications

1. Dispositif d'obtention d'un échantillon de sang (18), le dispositif comprenant :
un support (12) pour recevoir une source d'échantillon, le support (12) ayant une partie d'actionnement (24) et un orifice (26) ;
une partie d'engagement de récipient (56) reliée au support (12) ; et
un récipient de collecte (16) pouvant être relié de manière amovible à la partie d'engagement de récipient (56) ;
**caractérisé en ce que** la partie d'engagement de récipient (56) permet au récipient de collecte (16) de tourner entre une première position dans laquelle le récipient de collecte (16) est espacé de l'orifice (26) et une deuxième position dans laquelle le récipient de collecte (16) est en communication fluidique avec l'orifice (26).

2. Dispositif de la revendication 1, **caractérisé en ce que** la partie d'actionnement (24) peut effectuer une transition entre une première position dans laquelle le support (12) définit une première ellipse et une deuxième position dans laquelle le support (12) définit une deuxième ellipse, où la deuxième ellipse est différente de la première ellipse.

3. Dispositif de la revendication 1, **caractérisé en ce que** la partie d'actionnement (24) comprend un élément de contact (34).

4. Dispositif de la revendication 3, **caractérisé en ce que** la partie d'actionnement (24) peut effectuer une transition entre une première position dans laquelle l'élément de contact (34) est dans une position désengagée et une deuxième position dans laquelle l'élément de contact (34) est dans une position engagée.

5. Dispositif de la revendication 4, **caractérisé en ce que**, avec l'élément de contact (34) en position engagée, l'élément de contact (34) exerce une pression sur la source d'échantillon.

6. Dispositif de la revendication 1, **caractérisé en ce que** la partie d'actionnement (24) comprend un élément de pompage (36) pour appliquer une pression à la source d'échantillon.

7. Dispositif de la revendication 6, **caractérisé en ce que** l'élément de pompage (36) comprend une paire de pattes opposées (38).

8. Dispositif de la revendication 1, **caractérisé en ce que** la source d'échantillon est un doigt (19).

9. Dispositif de la revendication 8, **caractérisé en ce que**, avec le doigt (19) reçu à l'intérieur du support (12), l'orifice (26) est en communication avec une partie du doigt (19).

10. Dispositif de la revendication 1, **caractérisé en ce que** la partie d'engagement de récipient (56) et le support (12) sont reliés par l'intermédiaire d'un joint à rotule, d'un joint pivotant à cheville et trou, d'un joint pivotant à goupille et clip en C, ou d'un joint pivotant à goupille et poche.

11. Dispositif de la revendication 1, **caractérisé en ce que** le support (12) comprend une charnière mobile (186) qui peut être reliée à la partie d'engagement de récipient (56).

12. Dispositif de la revendication 11, **caractérisé en ce que** la charnière mobile (186) est reliée à la partie d'engagement de récipient (56) par l'intermédiaire d'un agencement à encliquetage claveté, d'un agencement à crochet et à encliquetage, d'un agencement à clip en C et à encliquetage, ou d'un engagement profilé et à cheville.

13. Dispositif de la revendication 1, **caractérisé en ce que** le récipient définit une cavité de collecte (70).
